# EUROPEAN PATENT SPECIFICATION

(11) **EP 3 326 594 B1**
(45) Date of publication and mention of the grant of the patent: **20.03.2019**
(21) Application number: 15898945.9
(22) Date of filing: 23.07.2015
(51) Int. Cl.: A61F 13/15, A61F 13/49, A61F 13/496, B65H 45/22

(54) **PRODUCTION APPARATUS FOR BIFOLDED MEMBER FOR ABSORBENT ARTICLES AND PRODUCTION METHOD**
HERSTELLUNGSVORRICHTUNG FÜR DOPPELFALTUNGSELEMENT FÜR SAUGFÄHIGE ARTIKEL UND HERSTELLUNGSVERFAHREN
APPAREIL DE PRODUCTION D'UN ÉLÉMENT PLIÉ EN DEUX DESTINÉ À DES ARTICLES ABSORBANTS ET PROCÉDÉ DE PRODUCTION

(43) Date of publication of application: 30.05.2018
(73) Proprietor: Unicharm Corporation, Shikokuchuo-shi, Ehime 799-0111 (JP)
(72) Inventor: MAITANI, Mitsuo, Kanonji-shi Kagawa 769-1602 (JP); HAMADA, Akira, Kanonji-shi Kagawa 769-1602 (JP)
(74) Representative: Staeger & Sperling Partnerschaftsgesellschaft mbB
(86) International application number: PCT/JP2015/070964
(87) International publication number: WO 2017/013786

(56) References cited:
- EP-A1- 2 550 944
- WO-A1-2008/142946
- CN-A- 104 337 622
- JP-A- 2010 227 545
- JP-A- 2011 200 385
- JP-A- 2012 525 286
- JP-A- 2013 519 402
- US-A1- 2002 174 930
- US-A1- 2010 050 411

## Description

### [Technical Field]

The present invention relates to a manufacturing apparatus of a two-folded member associated with an absorbent article such as a disposable diaper, and a method for manufacturing the two-folded member associated with the absorbent article.

### [Background Art]

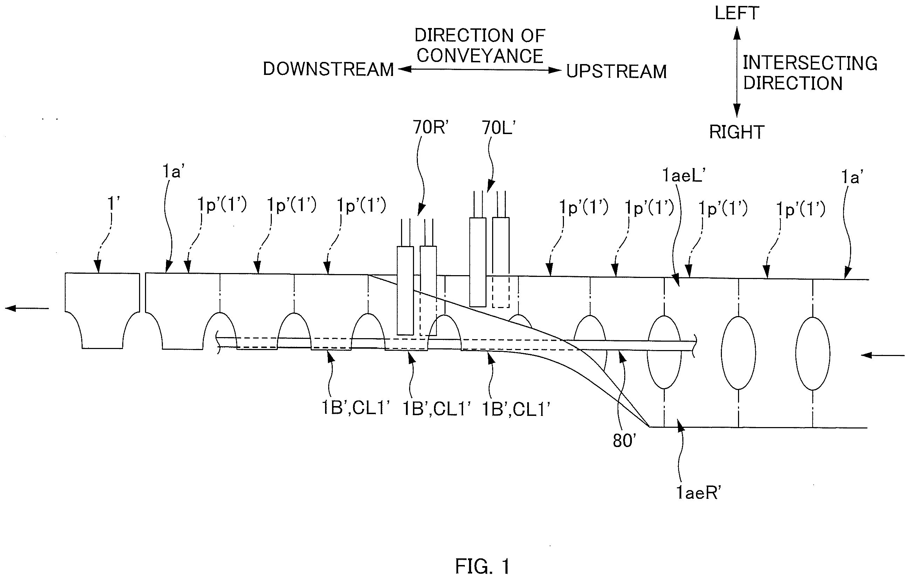

A disposable diaper 1' has been conventionally known as an absorbent article that absorbs excreted liquid such as urine. The diaper 1' is manufactured in a manufacturing line as illustrated in a schematic plan view in Fig. 1. In the manufacturing line, a continuous member 1a' includes a plurality of portions 1p' to be formed in a diaper 1' aligned in a continuous direction, and the continuous member 1a' is folded in two, thus manufacturing a two-folded member 1a' that is continuous in the continuous direction as an intermediate product 1a'. In other words, the above-described continuous member 1a' is folded with a certain portion CL1' in an intersecting direction intersecting with the continuous direction as a folding portion 1B', so as to fold the continuous member 1a' in two in the intersecting direction, thus manufacturing the above-described two-folded member 1a'.

Here, in the folding, one end portion 1aeL' and another end portion 1aeR' of the continuous member 1a' in the intersecting direction are put together. At this time, equipment disclosed in PTL 1 uses a position adjustment mechanism 70L' to adjust a position of the one end portion 1aeL' in the intersecting direction so as to be positioned at a target position, while using another position adjustment mechanism 70R' to adjust a position of the other end portion 1aeR' in the intersecting direction so as to be positioned at a target position.

Also, in the folding, a rod-shaped guiding member 80' is disposed to abut against the folding portion 1B' from a valley side. This restricts the movement of the folding portion 1B' in the intersecting direction, while allowing the folding portion 1B' to move in a direction of conveyance, thus achieving stabilization of the movement of the folding portion 1B' in the direction of conveyance.

### [Citation List]

### [Patent Literature]

[PTL 1] Japanese Unexamined Patent Application Publication No. 2005-46246

### [Summary of Invention]

### [Technical Problem]

However, the guiding member 80' is a rod-shaped member 80' unmovably fixed in the manufacturing line in the direction of conveyance. Accordingly, a large sliding resistance to the folding portion 1B' moving in the direction of conveyance possibly occurs, thus causing a delay of the movement of the folding portion 1B' in the direction of conveyance to a relatively upstream side in the direction of conveyance compared with other portions of the continuous member 1a'. As a result, deterioration of manufacturing accuracy of the diaper 1' possibly occurs, for example, the folding portion 1B' is fixed by welding and the like while being in an inclined state with respect to a position to be originally positioned.

Here, the sliding resistance easily increases at positions in the direction of conveyance where the position adjustment mechanisms 70L' and 70R' are disposed, for example. That is, when the position adjustment mechanisms 70L' and 70R' move the one end portion 1aeL' or the other end portion 1aeR' in the intersecting direction, the folding portion 1B' sometimes strongly contacts the guiding member 80', and in this case, the sliding resistance increases compared with the other positions in the direction of conveyance. Thus, the large sliding resistance that possibly occurs on this position is likely to be one of main causes for the deterioration of the manufacturing accuracy of the above-mentioned diaper 1'.

The present invention has been made in consideration of the conventional problems as described above. An object of the present invention is to reduce increase in sliding resistance between a folding portion and a guiding member, the sliding resistance possibly occurring in accordance with an adjustment of a position of an end portion of a continuous member in the intersecting direction so that the end portion is positioned at a target position when the continuous member is folded in two in the intersecting direction.

### [Solution to Problem]

A main aspect of the invention for achieving the above objective is a manufacturing apparatus of a two-folded member associated with an absorbent article according to claim 1, the manufacturing apparatus manufacturing two-folded members continuous in a continuous direction by folding at a folding portion a continuous member including a plurality of portions serving as absorbent articles aligned in the continuous direction so that the continuous member is folded in two in an intersecting direction, the folding portion being a predetermined portion in the intersecting direction intersecting with the continuous direction, the manufacturing apparatus including:
a folding mechanism configured to fold the continuous member at the folding portion so as to place one end portion on another end portion of the continuous member in the intersecting direction, the continuous member being conveyed in the continuous direction as a direction of conveyance;
a holding mechanism configured to hold the one end portion of the continuous member so that the one end portion is positioned at a proper position in the intersecting direction;
a guiding member configured to guide the folding portion in such a manner that the guiding member abuts against the continuous member to be folded from a valley side of the folding portion so as to restrict a movement of the folding portion in the intersecting direction while allowing a movement of the folding portion in the direction of conveyance; and
a position adjustment mechanism configured to adjust a position of the other end portion in such a manner that the position adjustment mechanism imparts an external force in the intersecting direction at a predetermined position in the direction of conveyance to the other end portion of the continuous member to be folded so as to position the other end portion at a target position in the intersecting direction,
an endless movable guiding member as the guiding member being disposed at least at the predetermined position in the direction of conveyance to which the external force is imparted, the endless movable guiding member moving downstream in the direction of conveyance while abutting against the continuous member from the valley side of the folding portion,
   a non-installation region where the movable guiding member is not installed existing on a conveyance path of the continuous member,
the non-installation region being provided with an immovable guiding member as the guiding member, the immovable guiding member being immovably fixed in the direction of conveyance and the intersecting direction and guiding the folding portion in the direction of conveyance,
the movable guiding member being disposed downstream in the direction of conveyance with respect to the immovable guiding member.

Further, another main aspect of the invention for achieving the above objective is a manufacturing method of a two-folded member associated with absorbent article according to claim 9, the manufacturing method manufacturing two-folded members continuous in a continuous direction by folding at a folding portion a continuous member including a plurality of portions serving as absorbent articles aligned in the continuous direction so that the continuous member is folded in two in an intersecting direction, the folding portion being a predetermined portion in the intersecting direction intersecting with the continuous direction, the manufacturing method including:
placing one end portion on another end portion of the continuous member in the intersecting direction by allowing a folding mechanism to fold the continuous member at the folding portion, the continuous member being conveyed in the continuous direction as a direction of conveyance;
holding the one end portion of the continuous member by a holding mechanism so that the one end portion is positioned at a proper position in the intersecting direction;
guiding the folding portion in such a manner that a guiding member abuts against the continuous member to be folded from a valley side of the folding portion so as to restrict a movement of the folding portion in the intersecting direction while allowing a movement of the folding portion in the direction of conveyance; and
adjusting a position of the other end portion in such a manner that a position adjustment mechanism imparts an external force in the intersecting direction at a predetermined position in the direction of conveyance to the other end portion of the continuous member to be folded so as to position the other end portion at a target position in the intersecting direction,
at least at the predetermined position in the direction of conveyance to which the external force is imparted, an endless movable guiding member as the guiding member moving downstream in the direction of conveyance while abutting against the continuous member from the valley side of the folding portion,
   guiding the folding portion in the direction of conveyance in a non-installation region, wherein the non-installation region is a region where the movable guiding member is not installed existing on a conveyance path of the continuous member,
the non-installation region being provided with an immovable guiding member as the guiding member, the immovable guiding member being immovably fixed in the direction of conveyance and the intersecting direction and guiding the folding portion (1B) in the direction of conveyance,
the movable guiding member being disposed downstream in the direction of conveyance with respect to the immovable guiding member.

Other features of the present invention will be made clear by the description and attached drawings.

### [Advantageous Effects of Invention]

According to the present invention, the increase in sliding resistance between a folding portion and a guiding member can be reduced, the sliding resistance possibly occurring in accordance with an adjustment of a position of an end portion of a continuous member in the intersecting direction so that the end portion is positioned on a target position when the continuous member is folded in two in the intersecting direction.

### [Brief Description of Drawings]

Fig. 1 is a schematic plan view illustrating a manufacturing process of a diaper 1'.
Fig. 2 is a schematic perspective view of a three-piece type diaper 1 as an example of a disposable diaper 1.
Fig. 3 is a schematic plan view of the diaper 1 in its opened state viewed from a skin side.
Fig. 4 is a cross-sectional view taken along the line IV-IV in Fig. 3.
Fig. 5 is a schematic perspective view of a manufacturing apparatus 30 for a two-folded member 1a associated with an absorbent article 1 of the present embodiment.
Fig. 6A is a schematic plan view, and Fig. 6B is a schematic side view taken along arrows B-B in Fig. 6A.
Fig. 7 is a schematic perspective view of the manufacturing apparatus 30 without illustrating an intermediate product 1a.
Fig. 8A is a schematic plan view, and Fig. 8B is a schematic side view taken along the arrows B-B in Fig. 8A.
Fig. 9A is a schematic plan view illustrating an enlarged position adjustment mechanism 70, and Fig. 9B is a schematic side view taken along the arrows B-B in Fig. 9A.
Fig. 10 is a schematic perspective view of a manufacturing apparatus 30 in a first modification.
Fig. 11A is a schematic plan view of a manufacturing apparatus 30 in a second modification, and Fig. 11B is a schematic side view taken along the arrows B-B in Fig. 11A.

### [Description of Embodiments]

At least the following matters are made clear from the description and drawings described below.

Disclosed is a manufacturing apparatus of a two-folded member associated with an absorbent article, the manufacturing apparatus manufacturing two-folded members continuous in a continuous
direction by folding at a folding portion a continuous member including a plurality of portions serving as absorbent articles aligned in the continuous direction so that the continuous member is folded in two in an intersecting direction, the folding portion being a predetermined portion in the intersecting direction intersecting with the continuous direction, the manufacturing apparatus including:
a folding mechanism configured to fold the continuous member at the folding portion so as to place one end portion on another end portion of the continuous member in the intersecting direction, the continuous member being conveyed in the continuous direction as a direction of conveyance;
a holding mechanism configured to hold the one end portion of the continuous member so that the one end portion is positioned at a proper position in the intersecting direction;
a guiding member configured to guide the folding portion in such a manner that the guiding member abuts against the continuous member to be folded from a valley side of the folding portion so as to restrict a movement of the folding portion in the intersecting direction while allowing a movement of the folding portion in the direction of conveyance; and
a position adjustment mechanism configured to adjust a position of the other end portion in such a manner that the position adjustment mechanism imparts an external force in the intersecting direction at a predetermined position in the direction of conveyance to the other end portion of the continuous member to be folded so as to position the other end portion at a target position in the intersecting direction,
an endless movable guiding member as the guiding member being disposed at least at the predetermined position in the direction of conveyance to which the external force is imparted, the endless movable guiding member moving downstream in the direction of conveyance while abutting against the continuous member from the valley side of the folding portion..

According to such a manufacturing apparatus of the two-folded member associated with the absorbent article, the movable guiding member as the guiding member having the above-described function is disposed at the predetermined position in the direction of conveyance where the external force in the intersecting direction is imparted to the other end portion to adjust the position of the other end portion of the continuous member. The movable guiding member moves downstream in the direction of conveyance while abutting against the continuous member from the valley side of the folding portion. Accordingly, sliding resistance that possibly occurs in the folding portion of the continuous member can be reduced. As a result, increase in the sliding resistance between the folding portion of the continuous member and the guiding member, which possibly occurs in accordance with the adjustment of the position of the other end portion of the continuous member, can be reduced.

In the manufacturing apparatus of the two-folded member associated with the absorbent article, a non-installation region where the movable guiding member is not installed exists on a conveyance path of the continuous member, and the non-installation region is provided with an immovable guiding member as the guiding member which is immovably fixed in the direction of conveyance and the intersecting direction and guides the folding portion in the direction of conveyance.

According to such a manufacturing apparatus of the two-folded member associated with the absorbent article, while the immovable guiding member is disposed in the non-installation region where the movable guiding member is not installed, the immovable guiding member is achievable with a simple configuration, for example, a rod-shaped member is sufficient. Accordingly, stability of the conveyance of the folding portion can be enhanced over a wider range of the conveyance path at a low price.

In the manufacturing apparatus of the two-folded member associated with the absorbent article, the movable guiding member is disposed downstream in the direction of conveyance with respect to the immovable guiding member.

According to such a manufacturing apparatus of the two-folded member associated with the absorbent article, the movable guiding member is disposed downstream in the direction of conveyance with respect to the immovable guiding member. Accordingly, it is possible to deal with a tendency that the sliding resistance easily increases in a downstream side compared with an upstream side in the direction of conveyance without any serious problem. That is, since a folded degree of the folding portion of the continuous member increases as the folding portion moves downstream in the direction of conveyance, a portion of the continuous member, which abuts against the guiding member, gradually increases, and as a result, the sliding resistance tends to increase in the downstream side compared with the upstream side in the direction of conveyance. However, in this regard, the movable guiding member is disposed downstream in the direction of conveyance with respect to the immovable guiding member. Accordingly, the sliding resistance that possibly increases in the downstream side compared with the upstream side in the direction of conveyance can be reduced.

In the manufacturing apparatus of the two-folded member associated with the absorbent article, it is preferable that the continuous member includes a plurality of absorbent main bodies aligned at intervals between the absorbent main bodies adjacent in the direction of conveyance, the folding portion is positioned in the absorbent main body,
an unguidable section in which both the immovable guiding member and the movable guiding member are not able to guide the folding portion exists at a boundary position between the immovable guiding member and the movable guiding member in the direction of conveyance, and
the unguidable section has a length in the direction of conveyance configured to be shorter than a length of the absorbent main body in the direction of conveyance at a position of the folding portion.

According to such a manufacturing apparatus of the two-folded member associated with the absorbent article, the unguidable section has the length in the direction of conveyance configured to be shorter than the length of the absorbent main body in the direction of conveyance at the position of the folding portion. Thus, when the absorbent main body passes through the unguidable section, the absorbent main body transfers from the immovable guiding member to the movable guiding member through a state in which the absorbent main body is bridged on both the immovable guiding member and the movable guiding member. Accordingly, the transfer can be smoothly performed compared with a case where the transfer is performed through a state where the absorbent main body is not guided by either the immovable guiding member or the movable guiding member.

In the manufacturing apparatus of the two-folded member associated with the absorbent article, it is preferable that the continuous member includes a plurality of absorbent main bodies aligned at intervals between the absorbent main bodies adjacent in the direction of conveyance, the folding portion is positioned in the absorbent main body,
a pressing device is disposed downstream in the direction of conveyance with respect to the folding mechanism, the pressing device is configured to convey the continuous member in a two-folded state in the direction of conveyance while sandwiching and pressing the continuous member in a thickness direction of the continuous member, and
a length in the direction of conveyance of an interval between a guide termination position and a pressing start position is shorter than the length of the absorbent main body in the direction of conveyance at the position of the folding portion, the guide termination position being a position where the movable guiding member terminates the guide of the folding portion in the direction of conveyance, the pressing start position being a position where the pressing device starts pressing the continuous member.

According to such a manufacturing apparatus of the two-folded member associated with the absorbent article, the length in the direction of conveyance of the interval between the guide termination position and the pressing start position is configured to be shorter than the length of the absorbent main body in the direction of conveyance at the position of the folding portion. Thus, when the absorbent main body passes through the interval, the absorbent main body transfers from the movable guiding member to the pressing device through a state where the absorbent main body is bridged on both the movable guiding member and the pressing device. Accordingly, the transfer can be smoothly performed compared with a case where the transfer is performed through a state where the absorbent main body is not guided by either the movable guiding member or the pressing device.

In the manufacturing apparatus of the two-folded member associated with the absorbent article, it is preferable that a plurality of the movable guiding members are disposed to be aligned in the direction of conveyance.

According to such a manufacturing apparatus of the two-folded member associated with the absorbent article, the plurality of the movable guiding members are disposed to be aligned in the direction of conveyance. Therefore, the length of each movable guiding member can be shortened. Accordingly, the problems such as a slack of the movable guiding member that possibly occurs when the length of each movable guiding member is long can be effectively prevented, and as a result, each movable guiding member can more certainly guide the folding portion of the continuous member.

In the manufacturing apparatus of the two-folded member associated with the absorbent article, it is preferable that the movable guiding member guides the folding portion over a range greater than or equal to 70% of an arrangement range of the folding mechanism in the direction of conveyance.

According to such a manufacturing apparatus of the two-folded member associated with the absorbent article, the folding portion is guided by the movable guiding member over the range equal to or more than 70% of the arrangement range of the folding mechanism. Accordingly, in the state where the sliding resistance of the folding portion is reduced, this folding portion can be guided over a long range in the direction of conveyance.

In the manufacturing apparatus of the two-folded member associated with the absorbent article, it is preferable that the continuous member includes a plurality of absorbent bodies aligned at intervals between the absorbent bodies adjacent in the direction of conveyance,
a pressing device is disposed downstream in the direction of conveyance with respect to the folding mechanism, the pressing device configured to convey the continuous member in the two-folded state in the direction of conveyance while pressing the continuous member in a thickness direction of the continuous member,
the pressing device includes a pair of endless belts configured to be driven to go around along the direction of conveyance,
a conveyance path of the continuous member in the two-folded state is formed between the pair of the endless belts, the continuous member is sandwiched and pressed by the pair of the endless belts when the continuous member passes through the conveyance path, and
an outer peripheral surface of each of the endless belts includes a portion against which the continuous member abuts, the portion has non-stickiness, and each of the endless belts has an elastic deformability in a thickness direction of each of the endless belts, the elastic deformability being able to sandwich and press a portion without the absorbent body in the continuous member.

According to such a manufacturing apparatus of the two-folded member associated with the absorbent article, an outer peripheral surface of the endless belt of the pressing device includes a portion against which the continuous member abuts, and the portion has non-stickiness. This can suppress the adhesive that possibly exudes from the continuous member when pressed from attaching to the outer peripheral surface of this endless belt, and as a result, the problems such as contaminations of the endless belt and attachment of the continuous member to this belt can be effectively prevented.

Moreover, this endless belt has the above-mentioned level of elastic deformability. Accordingly, on the portion of the outer peripheral surface of the endless belt that faces the absorbent body at the time of the above-described pressing, the endless belt can be significantly depressed promptly in the thickness direction. Thus, the endless belt can sufficiently press not only a portion of the continuous member where the absorbent body exists, but also a portion where the absorbent body does not exist. As a result, the continuous member can be certainly sandwiched and pressed over the substantially entire region thereof.

In the manufacturing apparatus of the two-folded member associated with the absorbent article, it is preferable that the manufacturing apparatus includes a second position adjustment mechanism configured to adjust a position of the one end portion in such a manner that the second position adjustment mechanism imparts an external force in the intersecting direction to the one end portion of the continuous member at a second predetermined position in the direction of conveyance so as to position the one end portion at a target position in the intersecting direction, and
the movable guiding member that moves downstream in the direction of conveyance while abutting against the continuous member from the valley side of the folding portion is provided also at the second predetermined position.

According to such a manufacturing apparatus of the two-folded member associated with the absorbent article, the above-mentioned movable guiding member is disposed also at the second predetermined position in the direction of conveyance where the external force in the intersecting direction is imparted to the one end portion of the continuous member so as to adjust the position of the one end portion. Then, the movable guiding member moves downstream in the direction of conveyance while abutting against the continuous member from the valley side of the folding portion. Accordingly, even at the second predetermined position, the sliding resistance that possibly occurs on the folding portion of the continuous member can be reduced, and as a result, the increase in the sliding resistance between the folding portion of the continuous member and the guiding member, which possibly occurs in accordance with the adjustment of the position of the one end portion of the continuous member, can be reduced.

In the manufacturing apparatus of the two-folded member associated with the absorbent article, it is preferable that the second position adjustment mechanism functions as the holding mechanism, and
a suction belt conveyor configured to hold the one end portion of the continuous member is not disposed.

According to such a manufacturing apparatus of the two-folded member associated with the absorbent article, since the suction belt conveyor is not disposed, the facility cost can be significantly reduced.

Further disclosed is a manufacturing method of a two-folded member associated with absorbent article, the manufacturing method manufacturing two-folded members continuous in a continuous direction by folding at a folding portion a continuous member including a plurality of portions serving as absorbent articles aligned in the continuous direction so that the continuous member is folded in two in an intersecting direction, the folding portion being a predetermined portion in the intersecting direction intersecting with the continuous direction, the manufacturing method including:
placing one end portion on another end portion of the continuous member in the intersecting direction by allowing a folding mechanism to fold the continuous member at the folding portion, the continuous member being conveyed in the continuous direction as a direction of conveyance;
holding the one end portion of the continuous member by a holding mechanism so that the one end portion is positioned at a proper position in the intersecting direction;
guiding the folding portion in such a manner that a guiding member abuts against the continuous member to be folded from a valley side of the folding portion so as to restrict a movement of the folding portion in the intersecting direction while allowing a movement of the folding portion in the direction of conveyance; and
adjusting a position of the other end portion in such a manner that a position adjustment mechanism imparts an external force in the intersecting direction at a predetermined position in the direction of conveyance to the other end portion of the continuous member to be folded so as to position the other end portion at a target position in the intersecting direction,
at least at the predetermined position in the direction of conveyance to which the external force is imparted, an endless movable guiding member as the guiding member moving downstream in the direction of conveyance while abutting against the continuous member from the valley side of the folding portion.

According to such a manufacturing method for the two-folded member associated with the absorbent article, effects similar to the case of the above-mentioned manufacturing apparatus can be provided.

### === Present Embodiment ===

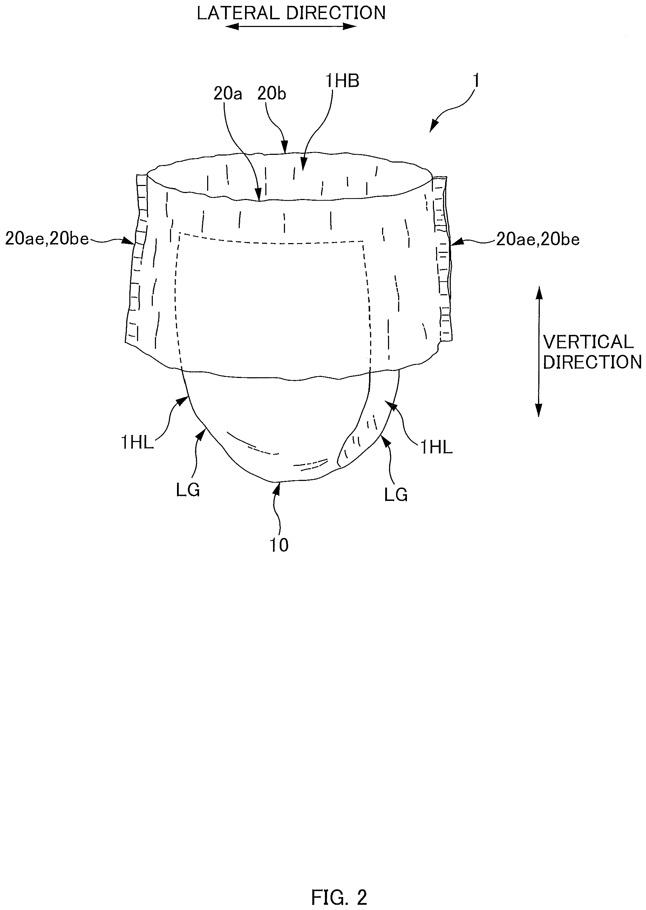

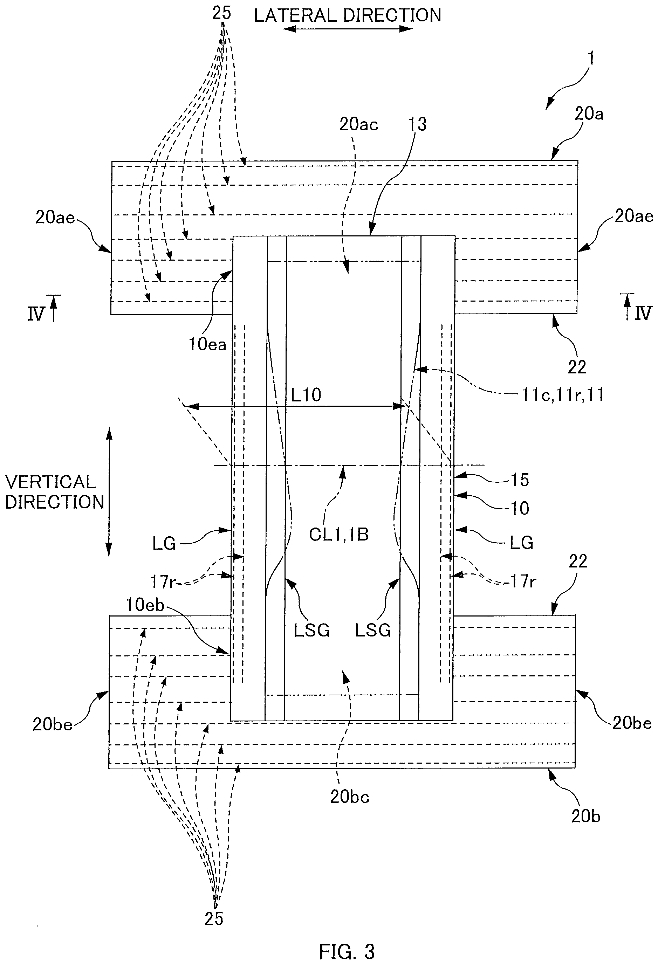

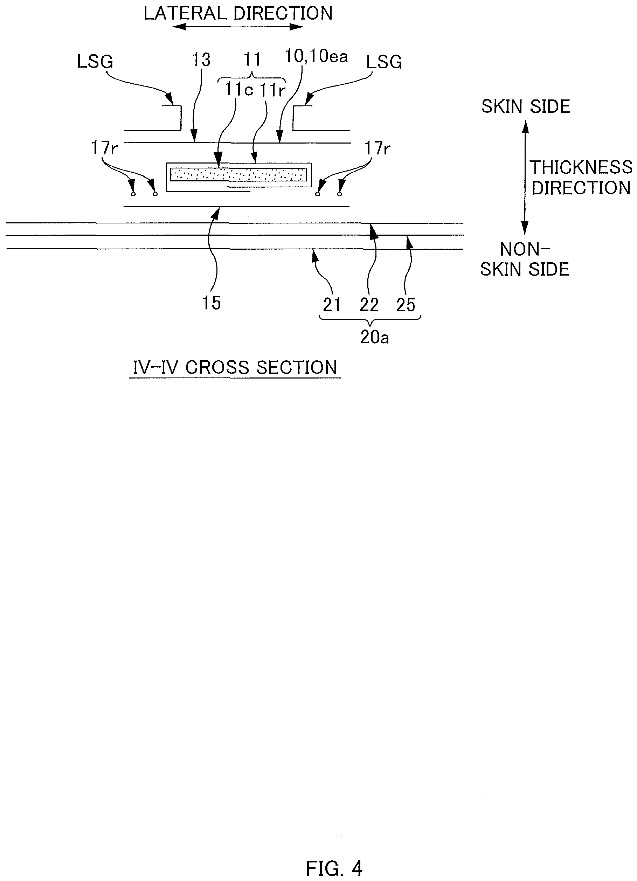

A manufacturing apparatus 30 and a manufacturing method for manufacturing a two-folded member 1a associated with an absorbent article 1 of the present embodiment are used, for example, in a manufacturing line of a disposable diaper 1 as an example of an absorbent article. Fig. 2 is a schematic perspective view of a three-piece type diaper 1 as an example of this disposable diaper 1. Fig. 3 is a schematic plan view of the diaper 1 in its opened state viewed from a skin side. Fig. 4 is a cross-sectional view taken along line IV-IV in Fig. 3.

In the opened state of Fig. 3 and Fig. 4, this diaper 1 has a vertical direction, a lateral direction, and a thickness direction as three directions that are mutually orthogonal. Further, this diaper 1 is a so-called three-piece type diaper, thus including a first component, a second component, and a third component. The first component is an absorbent main body 10 that is longitudinally long and is mainly placed on a crotch part of a wearer to absorb bodily waste such as urine. The second component is a front band member 20a that is laterally long and mainly covers a body of this wearer from a front. The third component is a back band member 20b that is laterally long and mainly covers the body of this wearer from a back.

Then, in the opened state in Fig. 3, in a state where the front band member 20a and the back band member 20b are disposed to be parallel with an interval between each another in the vertical direction, the absorbent main body 10 is bridged between them in the vertical direction, and end portions 10ea and 10eb of the absorbent main body 10 in the vertical direction are each bonded and fixed to respective nearest band members 20a and 20b by a hot-melt adhesive, thus forming an appearance shape in an approximately H shape in a plan view. From this state, the diaper 1 is folded in two with an approximately center portion CL1 (corresponding to a certain portion) of the absorbent main body 10 in the vertical direction as a folding portion 1B. When the band members 20a and 20b, which are opposed to one another in this two-folded state, are coupled by welding or the like at portions 20ae and 20be (that is, end portions 20ae and 20be in the lateral direction) configured to abut on flanks of a target wearer, the band members 20a and 20b are coupled to one another in a ring shape, thereby forming the diaper 1 in a pull-on state where a waist opening 1HB and a pair of leg openings 1HL, 1HL are formed as illustrated in Fig. 2. The following describes the respective elements 10, 20a, and 20b of this diaper 1.

The absorbent main body 10 is a sheet-shaped member which is longitudinally long and has an approximately rectangular shape when seen in a plan view. As illustrated in Fig. 3 and Fig. 4, the absorbent main body 10 includes an absorbent body 11, a liquid permeable front side sheet 13 that covers the absorbent body 11 from the skin side in the thickness direction to form a skin side surface of the absorbent main body 10, and a liquid non-permeable back side sheet 15 that covers the absorbent body 11 from a non-skin side in the thickness direction to form a non-skin side surface of the absorbent main body 10. The absorbent body 11 includes an absorbent core 11c obtained by forming liquid absorbent material such as pulp fiber and superabsorbent polymer into a predetermined shape such as an approximately hourglass shape when seen in a plan view, and a liquid permeable core-wrapping sheet 11r that coats this core 11c.

In this example, as illustrated in Fig. 3 and Fig. 4, the absorbent main body 10 includes barrier cuffs LSG, LSG as leak-proof walls on respective end portions in the lateral direction to prevent side leakage. The absorbent main body 10 includes elastic members 17r such as an elastic string disposed on both side portions in the lateral direction, thus, as illustrated in Fig. 2, forming stretchable leg gathers LG, LG on portions corresponding to the leg openings 1HL, 1HL of the diaper 1. However, those may be omitted.

On the other hand, as illustrated in Fig. 3, the front band member 20a and the back band member 20b are each a sheet-shaped member that is laterally long and has an approximately rectangular shape when seen in a plan view, and as illustrated in Fig. 4, each includes an outer-layer sheet 21 and an inner-layer sheet 22 positioned on the skin side with respect to the outer-layer sheet 21 in the thickness direction. Then, between the outer-layer sheet 21 and the inner-layer sheet 22, a plurality of elastic strings 25, 25 ..., which are disposed in a stretched state in the lateral direction along the lateral direction, are aligned in the vertical direction to be fixed by the hot-melt adhesive, thus imparting elasticity in the lateral direction to the band members 20a and 20b based on contractile force of the elastic strings 25, 25 .... Both the outer-layer sheet 21 and the inner-layer sheet 22 are formed of nonwoven fabric made of thermoplastic resin fiber in this example. However, the outer-layer sheet 21 and the inner-layer sheet 22 are not limited thereto as long as they are soft sheets.

Then, as illustrated in Fig. 3, the end portions 10ea and 10eb of the absorbent main body 10 in the vertical direction are overlapped on and joined to central regions 20ac and 20bc of the band members 20a and 20b in the lateral direction from the skin side.

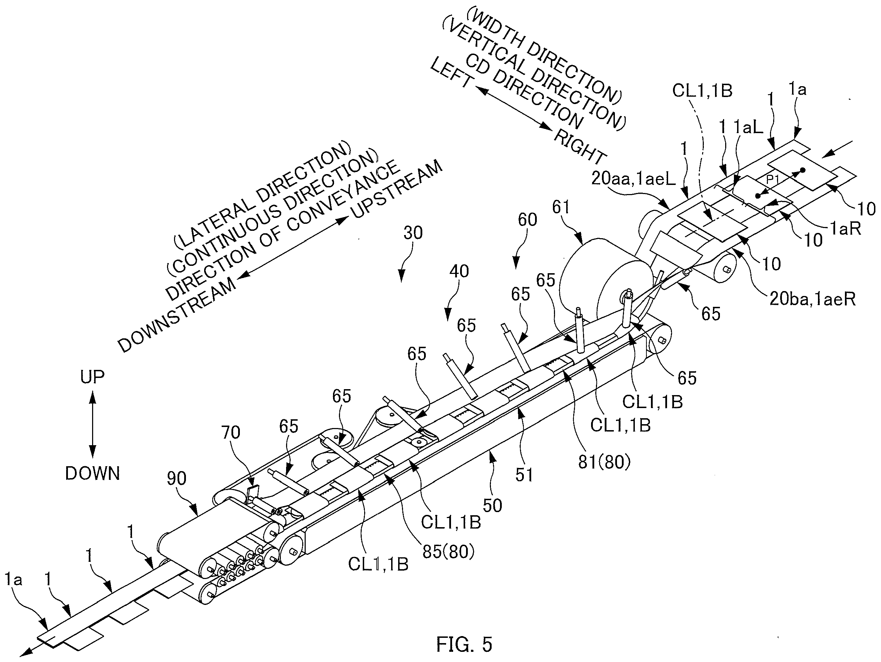

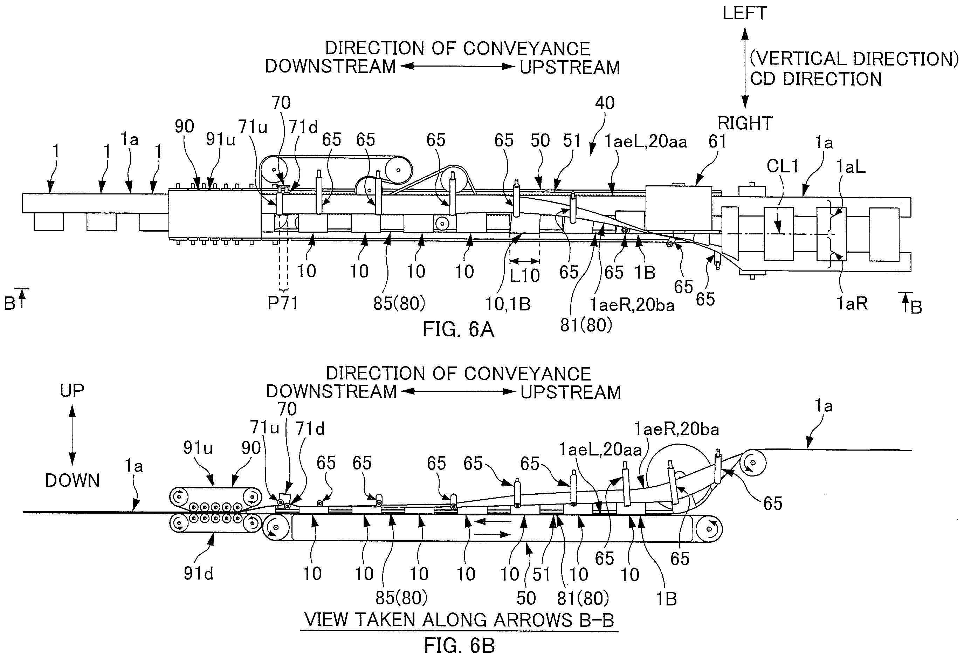

Fig. 5 is a schematic perspective view of the manufacturing apparatus 30 of the two-folded member 1a associated with the absorbent article 1 of the present embodiment. Fig. 6A is a schematic plan view, and Fig. 6B is a schematic side view taken along arrows B-B in Fig. 6A.

As illustrated in Fig. 5, the manufacturing apparatus 30 of the two-folded member 1a substantially manufactures an intermediate product 1a being in a state where a plurality of diapers 1, 1 ... in the pull-on state in Fig. 2 are aligned and coupled in the lateral direction, as an exemplary two-folded member 1a. That is, the intermediate product 1a (corresponding to the continuous member) immediately before being conveyed to the manufacturing apparatus 30 is a ladder-shaped continuous member 1a in which a plurality of diapers 1, 1 ... (corresponding to a "portion to be formed in the diaper") in the opened state of Fig. 3 are aligned and coupled in the lateral direction. More specifically, while the band members 20a and 20b are each in a form of continuous bodies 20aa and 20ba which are continuous in the lateral direction, a plurality of absorbent main bodies 10, 10 ... are aligned in the lateral direction at product pitches P1 and bridged in the vertical direction between the band member continuous bodies 20aa and 20ba. Then, while the ladder-shaped intermediate product 1a is conveyed in the continuous direction as the direction of conveyance, the manufacturing apparatus 30 folds the ladder-shaped intermediate product 1a with the approximately center portion CL1 (corresponding to the certain portion) as the folding portion 1B in the vertical direction perpendicular to the continuous direction, thus folding this intermediate product 1a in two in the vertical direction. As a result, the two-folded intermediate product 1a that is continuous in the direction of conveyance is manufactured.

In the following description, the vertical direction (corresponding to the intersecting direction) perpendicular to the direction of conveyance is also referred to as a "CD direction," and in this example, the CD direction faces a horizontal direction. One side in the CD direction is also referred to as a "right side," and the other side is also referred to as a "left side."

As illustrated in Fig. 5, the manufacturing apparatus 30 includes a two-folding device 40 and a pressing device 90 disposed on a downstream in the direction of conveyance with respect to the two-folding device 40. Then, the two-folding device 40 folds the ladder-shaped intermediate product 1a in two, and subsequently, the pressing device 90 sandwiches and presses the intermediate product 1a in the two-folded state in the thickness direction. Therefore, even in the following downstream process, the intermediate product 1a is easily maintained in the two-folded state. Consequently, a welding process, a cutting process and similar process can be performed to the intermediate product 1a in the two-folded state with high accuracy in the downstream process. The following describes the two-folding device 40 and the pressing device 90.

### <<<Two-folding device 40>>>

As illustrated in Fig. 5, the ladder-shaped intermediate product 1a is conveyed to the two-folding device 40 with a posture in which a width direction perpendicular to the continuous direction faces the CD direction. Then, as illustrated in Fig. 6A and Fig. 6B, in this device 40, the continuous body 20aa of the front band member forming a left end portion 1aeL (corresponding to one end portion) of the ladder-shaped intermediate product 1a in the CD direction is conveyed downstream in the direction of conveyance while maintaining the horizontal posture along the CD direction, whereas the continuous body 20ba of the back band member forming a right end portion 1aeR (corresponding to another end portion) in the CD direction is raised upward by folding the absorbent main body 10 with the approximately center portion CL1 of this main body 10 in the CD direction as the folding portion 1B. Then, the continuous body 20ba of this back band member is gradually laid toward the continuous body 20aa of the front band member, and finally, placed on the continuous body 20aa of the front band member. Accordingly, the ladder-shaped intermediate product 1a is changed into the intermediate product 1a in the two-folded state in which the continuous body 20aa of the front band member is placed on the continuous body 20ba of the back band member.

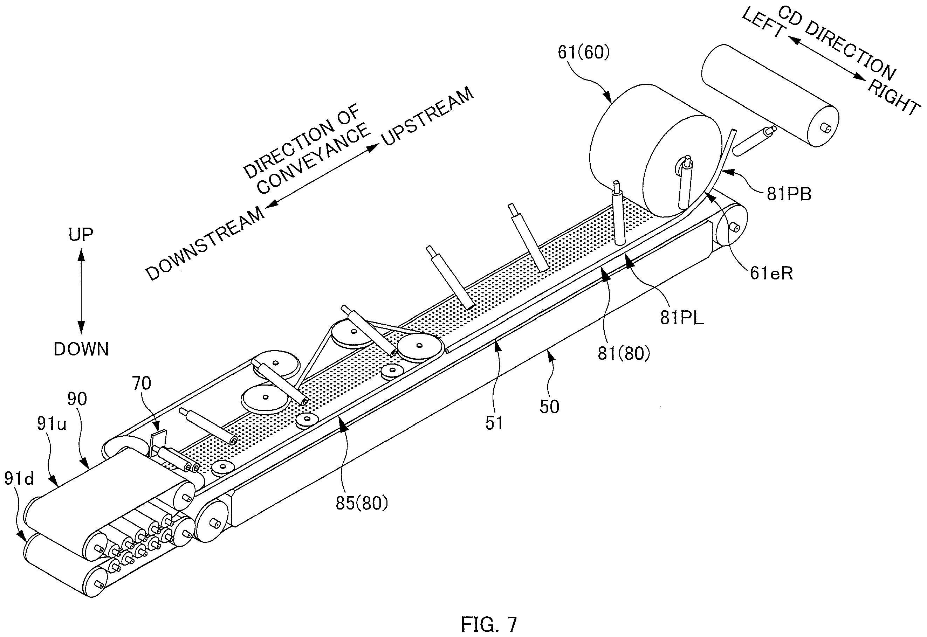

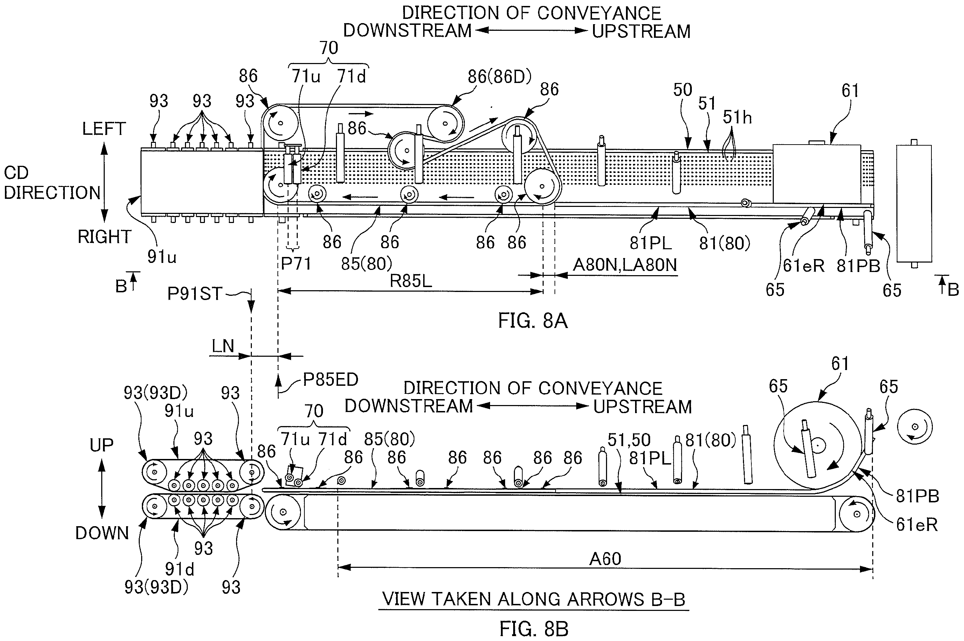

Fig. 7 is a schematic perspective view of the manufacturing apparatus 30 without illustrating the intermediate product 1a. Fig. 8A is a schematic plan view, and Fig. 8B is a schematic side view taken along the arrows B-B in Fig. 8A.

As illustrated in Fig. 5, the two-folding device 40 that performs the above-described two-folding process includes a holding mechanism 50, a folding mechanism 60, a position adjustment mechanism 70, and guiding members 80.

As illustrated in Fig. 6A, the holding mechanism 50 is a device configured to convey in the direction of conveyance the continuous body 20aa of the front band member forming the left end portion 1aeL of the ladder-shaped intermediate product 1a while holding this continuous body 20aa on a proper position in the CD direction, and in this example, a suction belt conveyor 50 is employed. That is, as illustrated in Fig. 8A, the conveyor 50 includes an endless belt 51 that is driven to go around along the direction of conveyance, and this belt 51 has an outer peripheral surface on which a plurality of suction holes 51h, 51h ... are formed. Accordingly, as illustrated in Fig. 6A and Fig. 6B, the continuous body 20aa of the front band member is adsorbed and held on the outer peripheral surface of the belt 51 from the lower side by suction of the suction holes 51h, 51h .... In this holding state, the belt 51 is driven to go around along the direction of conveyance in conjunction with a peripheral velocity value (m/sec) (for example, a peripheral velocity value in a range of ±5% of a conveyance velocity value, preferably a peripheral velocity value in a range of ±1% thereof) approximately identical to a conveyance velocity value (m/sec) of the ladder-shaped intermediate product 1a. Consequently, the holding mechanism 50 can hold the left end portion 1aeL of the intermediate product 1a on the proper position in the CD direction without inhibiting the conveyance state of the ladder-shaped intermediate product 1a.

As illustrated in Fig. 5, the folding mechanism 60 includes a rotating drum 61 and a plurality of guide rollers 65, 65 .... The rotating drum 61 has a large diameter, and is disposed on an upward position of the suction belt conveyor 50 and a position of an upstream end of this conveyor 50 in the direction of conveyance. The plurality of the guide rollers 65, 65 ... are disposed on upward positions of the conveyor 50, and discretely disposed on respective positions over an approximately whole length of the conveyor 50 in the direction of conveyance. Then, the rotating drum 61 is driven to rotate around a rotational shaft along the CD direction in conjunction with the peripheral velocity value (m/sec) (for example, the peripheral velocity value in the range of ±5% of the conveyance velocity value, preferably the peripheral velocity value in the range of ±1% thereof) approximately identical to the conveyance velocity value (m/sec) of the ladder-shaped intermediate product 1a. Accordingly, as illustrated in Fig. 5 and Fig. 6A, an outer peripheral surface of the rotating drum 61 first abuts from above on a left half portion 1aL of the ladder-shaped intermediate product 1a in the CD direction, thus pressing the left half portion 1aL with the outer peripheral surface. This raises a right half portion 1aR of the intermediate product 1a in cooperation with the near guide roller 65, thus making a starting point of the folding portion 1B on the approximately center portion CL1 of the ladder-shaped intermediate product 1a in the CD direction. After that, the raised right half portion 1aR is gradually laid toward the left half portion 1aL in the state of being held on this conveyor 50 by the guide rollers 65, 65 ... disposed on the respective positions in the direction of conveyance, and finally, the right half portion 1aR is laid to an approximately horizontal state. Then, this right half portion 1aR becomes in a state of being placed over the left half portion 1aL. In the state of being placed over, the continuous body 20ba of the back band member forming the right end portion 1aeR in the right half portion 1aR becomes in a state of being placed over the continuous body 20aa of the front band member forming the left end portion 1aeL on the left half portion 1aL.

Incidentally, the rotating drum 61 and the guide rollers 65, 65 ... are supported by appropriate supporting members disposed on the left side in the CD direction in the manufacturing line. Then, in this example, a so-called panel board (not illustrated) is employed as an example of the supporting members. The panel board is a plate member disposed upright on a floor portion of the manufacturing line, and this panel board has a vertical plane whose normal direction is directed to the CD direction. Then, the rotating drum 61 and the guide rollers 65, 65 ... are rotatably supported on the vertical plane via stay members (not illustrated) and the like in, for example, a cantilevered manner. However, the supporting member is not limited to the panel board, and a supporting member other than this may be employed.

As illustrated in Fig. 5, Fig. 6A, and Fig. 6B, the position adjustment mechanism 70 adjusts the position of the right end portion 1aeR in the CD direction at a position in the direction of conveyance where the right end portion 1aeR of the intermediate product 1a is placed over the left end portion 1aeL, or at a immediately close upstream position or immediately close downstream position. Accordingly, a displacement between the positions of the right end portion 1aeR and the left end portion 1aeL of the intermediate product 1a in the CD direction is decreased.

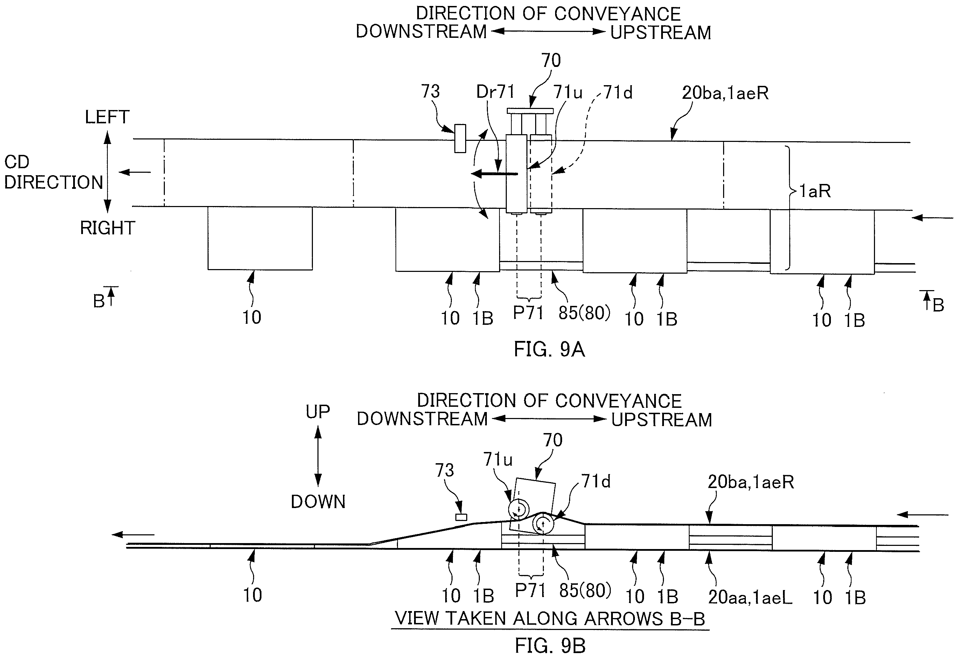

Fig. 9A is schematic plan view illustrating the enlarged position adjustment mechanism 70, and Fig. 9B is a schematic side view taken along arrows B-B in Fig. 9A.

As illustrated in Fig. 9A and Fig. 9B, the position adjustment mechanism 70 includes: for example, a pair of upper and lower rollers 71u and 71d disposed with their outer peripheral surfaces mutually opposed; an actuator (not illustrated) that drives to rotate the pair of the upper and lower rollers 71u and 71d within a plane having both the direction of conveyance and the CD direction; a sensor 73 such as a photoelectric tube disposed immediately downstream of the rollers 71u and 71d, and that measures the position of the right end portion 1aeR in the CD direction to output a measurement signal; and a controller (not illustrated) that controls the above-mentioned actuator based on the measurement signal from the sensor 73. Then, the continuous body 20ba of the back band member forming the right end portion 1aeR of the intermediate product 1a is disposed around the pair of the upper and lower rollers 71u and 71d in an S shape. Further, when the measurement signal indicates that the measured position is located on the left side with respect to the target position in the CD direction, the controller controls the actuator to direct a sending direction Dr71 of the pair of the upper and lower rollers 71u and 71d to the right side with respect to a current sending direction Dr71 in the CD direction, whereas when the measurement signal indicates that the measured position is located on the right side with respect to the target position in the CD direction, the controller controls the actuator to direct the sending direction of the pair of the upper and lower rollers 71u and 71d to the left side with respect to the current sending direction Dr71 in the CD direction. This is repeatedly performed at a predetermined control cycle, thus enabling the right end portion 1aR of the intermediate product 1a to be placed over the left end portion 1aL with high position accuracy.

As illustrated in Fig. 5, the guiding member 80 guides the folding portion 1B of the ladder-shaped intermediate product 1a in the direction of conveyance. That is, the guiding member 80 guides the folding portion 1B such that the guiding member 80 abuts against the ladder-shaped intermediate product 1a from a valley side of the folding portion 1B so as to restrict the movement of the folding portion 1B in the CD direction while allowing the movement of the folding portion 1B in the direction of conveyance. Then, as illustrated in Fig. 7, the guiding member 80 extends over a substantially entire length of the suction belt conveyor 50 in the direction of conveyance, thus maintaining the folding portion 1B on a fixed position in the CD direction during the two-folding process performed by the folding mechanism 60. As a result, stabilization of the two-folding process is achieved.

Here, in this embodiment, as illustrated in Fig. 7, Fig. 8A, and Fig. 8B, two kinds of guiding members 81 and 85 are disposed as the guiding member 80. That is, a rod-shaped immovable guiding member 81 immovably fixed in the direction of conveyance and the CD direction, and an endless movable guiding member 85 that moves downstream in the direction of conveyance are disposed. Then, the latter movable guiding member 85 is in conjunction with the conveyance operation of the ladder-shaped intermediate product 1a and moves downstream in the direction of conveyance at a velocity value (m/sec) (for example, a velocity value in a range of ±10% of the conveyance velocity value, preferably, a velocity value in a range of ±5% thereof, and more preferably, a velocity value in a range of ±1% thereof) approximately identical to the conveyance velocity value (m/sec). Accordingly, sliding resistance to the folding portion 1B illustrated in Fig. 5 can be reduced. Then, this prevents the movement of the absorbent main body 10 in the direction of conveyance which includes the folding portion 1B from being delayed to the upstream side relative to the movement of the continuous body 20aa of the front band member and the continuous body 20ba of the back band member in the direction of conveyance. Consequently, it is possible to prevent the deterioration in manufacturing accuracy of the diaper 1.

As illustrated in Fig. 8A and Fig. 9A, in this embodiment, the movable guiding member 85 is provided across an arrangement position P71 of the pair of the rollers 71u and 71d of the position adjustment mechanism 70 in the direction of conveyance, thus, even at the arrangement position P71, the movable guiding member 85 abuts against the folding portion 1B to guide it to the downstream side. Accordingly, the sliding resistance is effectively reduced at the arrangement position P71 where the sliding resistance possibly increases. Details are as follows.

First, as described above with reference to Fig. 5 and Fig. 6A, the left end portion 1aeL of the intermediate product 1a is held onto the proper position in the CD direction by the suction belt conveyor 50. Then, under this condition, as illustrated in Fig. 9A and Fig. 9B, when the pair of the rollers 71u and 71d of the position adjustment mechanism 70 gives an external force in the CD direction to the right end portion 1aeR to move this right end portion 1aeR in the CD direction, the folding portion 1B possibly strongly hit the guiding member 80. In this case, the sliding resistance between the folding portion 1B and the guiding member 80 possibly increases, and the movement in the direction of conveyance of the absorbent main body 10 on which the folding portion 1B is positioned may be relatively delayed to the upstream side compared with the continuous body 20aa of the front band member and the continuous body 20ba of the back band member. This may possibly deteriorate the manufacturing accuracy of the diaper 1. However, in this regard, in this embodiment, the movable guiding member 85 is disposed on the arrangement position P71 of the pair of the rollers 71u and 71d of the position adjustment mechanism 70 in the direction of conveyance, that is, on the position P71 in the direction of conveyance where the pair of the rollers 71u and 71d give the external force in the CD direction to the right end portion 1aeR of the intermediate product 1a,. Accordingly, at the position P71 (corresponding to the predetermined position), the movable guiding member 85 moves downstream in the direction of conveyance together with the folding portion 1B while abutting against the folding portion 1B. Thus, the movable guiding member 85 can guide the folding portion 1B in the direction of conveyance while significantly reducing the sliding resistance that possibly occurs in the folding portion 1B. This can prevent the deterioration in manufacturing accuracy of the above-described diaper 1.

In this example, a round belt 85 that has a cross section in a circular shape is employed as the movable guiding member 85. Then, as illustrated in Fig. 8A, the round belt 85 is disposed around a plurality of pulleys 86, 86 ... supported by a horizontal plate-shaped stay member (not illustrated), which projects out to the right side in the CD direction from the above-described panel board, so as to be driven to go around a predetermined approximately horizontal circulating track. That is, one of the plurality of the pulleys 86, 86 ... is a drive pulley 86D that obtains a rotation force from a driving source such as a servo motor (not illustrated) to be driven to rotate, and this causes the round belt 85 to be driven to go around. The circulating track includes a linear route R85L along the direction of conveyance on a right side position in the CD direction. Accordingly, the round belt 85 can smoothly guide in the direction of conveyance the folding portion 1B of the intermediate product 1a moving to the downstream side on the linear route R85L.

On the other hand, as illustrated in Fig. 8A and Fig. 8B, in this example, the immovable guiding member 81 that has a simple structure compared with the movable guiding member 85 is disposed in a region (corresponding to a non-installation region) on the upstream side in the direction of conveyance which is assumed that the sliding resistance is relatively small. This results in cost reduction. That is, as illustrated in Fig. 5, Fig. 6A, and Fig. 6B, as the folding portion 1B of the intermediate product 1a moves downstream in the direction of conveyance, a folded degree of the folding portion 1B increases, and this gradually increases portions of the intermediate product 1a that abut against the guiding member 80. Accordingly, the sliding resistance tends to increase on the downstream side compared with the upstream side in the direction of conveyance. Thus, on the upstream side in the direction of conveyance where the sliding resistance is small, the immovable guiding member 81 is applicable without problems, and this example employs such immovable guiding member 81.

As illustrated in Fig. 7 and Fig. 8B, the immovable guiding member 81 has a curved portion 81PB which is curved at a position of an upstream end in the direction of conveyance, and a linear portion 81PL along the direction of conveyance at a downstream position with respect to the curved portion 81PB. Then, the curved shape of the curved portion 81PB is an approximately arc shape upwardly displaced toward the upstream side, and the curved portion 81PB is disposed at the proximity of a right end edge 61eR of the rotating drum 61 in the CD direction associated with the above-described folding mechanism 60 along this end edge 61eR. Accordingly, the curved portion 81PB can promptly guide the starting point of the folding portion 1B formed cooperatively by the rotating drum 61 and the guide roller 65 in the vicinity thereof.

As illustrated in Fig. 7 and Fig. 8A, the linear portion 81PL is arranged at the right side position in the CD direction, and has a linear shape along the direction of conveyance. Accordingly, the linear portion 81PL can smoothly guide the folding portion 1B of the intermediate product 1a that is to be conveyed on an approximately linear conveyance path along the direction of conveyance after passing through the position of the rotating drum 61.

Now, in the example of Fig. 8A, on a boundary position between the immovable guiding member 81 and the movable guiding member 85 in the direction of conveyance, an unguidable section A80N where both the immovable guiding member 81 and the movable guiding member 85 cannot guide the folding portion 1B exists. Here, preferably, the unguidable section A80N has a length LA80N in the direction of conveyance configured to be shorter than a length L10 (see Fig. 3 or Fig. 6A) of the absorbent main body 10 in the direction of conveyance at the position of the folding portion 1B.

Then, in such a state, when each of the absorbent main bodies 10, 10 ... passes through the unguidable section A80N, the absorbent main body 10 transfers from the immovable guiding member 81 to the movable guiding member 85 through a state where the absorbent main body 10 is bridged on both the immovable guiding member 81 and the movable guiding member 85. Accordingly, the transfer can be smoothly performed compared with a case where the transfer is performed through a state where the absorbent main body 10 is not guided by either the immovable guiding member 81 or the movable guiding member 85.

### <<<Pressing device 90>>>

As illustrated in Fig. 8A and Fig. 8B, the pressing device 90 includes a pair of upper and lower endless belts 91u and 91d. The endless belts 91u and 91d are each disposed around a plurality of rollers 93, 93 ... rotatably supported around each rotation shaft along the CD direction. Then, one roller 93D among the plurality of the rollers 93, 93 ... is a drive roller that obtains a rotation force from a driving source such as a servo motor to rotate. Accordingly, the endless belts 91u and 91d are each driven to circle in the direction of conveyance. Between the pair of the upper and lower endless belts 91u and 91d, a conveyance path for the intermediate product 1a in the two-folded state is formed. Accordingly, as illustrated in Fig. 6B, when the intermediate product 1a in the two-folded state passes through the conveyance path, the pair of the endless belts 91u and 91d sandwich and press the intermediate product 1a in the thickness direction, thus easily maintaining this intermediate product 1a in the two-folded state even in downstream processes. This effectively contributes to the accuracy improvement of the welding process, the cutting process and the like performed on the intermediate product 1a in the two-folded state in downstream processes.

Here, in this example, as seen with reference to Fig. 6A, the intermediate product 1a in the two-folded state does not project outwardly in the CD direction from outer peripheral surfaces of the endless belts 91u and 91d, that is, the intermediate product 1a is disposed inside the outer peripheral surface in the CD direction. Accordingly, a pressing process can be performed on the entire region in the CD direction of the intermediate product 1a in the two-folded state.

The endless belts 91u and 91d have non-stickiness silicon rubber layers (not illustrated) over the entire region of portions on which the intermediate product 1a can abut in the outer peripheral surfaces. This can suppress the hot-melt adhesive that possibly exudes from the intermediate product 1a in the two-folded state when pressed from attaching to these endless belts 91u and 91d, and as a result, the problems such as contaminations of the endless belts 91u and 91d and attachment of the intermediate product 1a to these belts 91u and 91d can be effectively prevented.

Further, the endless belts 91u and 91d have excellent elastic deformability in the thickness direction based on the above-described silicon rubber layers. Accordingly, on portions of the outer peripheral surfaces of the endless belts 91u and 91d that face the absorbent body 11 at the time of above-described pressing, the endless belts 91u and 91d can be significantly depressed promptly in the thickness direction. Therefore, the endless belts 91u and 91d can sufficiently press not only a portion of the intermediate product 1a in the two-folded state where the absorbent body 11 exists, but also a portion where the absorbent body 11 does not exist, and as a result, the pressing process can be certainly performed over the entire region of the intermediate product 1a.

In this example, as illustrated in Fig. 8A and Fig. 8B, a length LN is configured to be shorter than the length L10 (see Fig. 3 or Fig. 6A) of the absorbent main body 10 in the direction of conveyance at the position of the folding portion 1B, the length LN being an interval in the direction of conveyance between a guide termination position P85ED where the movable guiding member 85 terminates the guide of the folding portion 1B in the direction of conveyance and a pressing start position P91ST where the endless belts 91u and 91d of the pressing device 90 start pressing the intermediate product 1a. Accordingly, when each of the absorbent main bodies 10, 10 ... passes through the interval, the absorbent main body 10 transfers from the movable guiding member 85 to the endless belts 91u and 91d of the pressing device 90 through a state where the absorbent main body 10 is bridged on both the movable guiding member 85 and the endless belts 91u and 91d of the pressing device 90. Accordingly, the transfer can be smoothly performed compared with a case where, for example, the absorbent main body transfers through a state where the absorbent main body 10 is not guided to either the movable guiding member 85 or the endless belts 91u and 91d of the pressing device 90. Incidentally, the above-described "guide termination position P85ED" is a downstream end position of the aforementioned linear route R85L in the direction of conveyance illustrated in Fig. 8A, and the above-described "pressing position P91ST" is a most upstream position among positions in the direction of conveyance where the intermediate product 1a abuts against both belts 91u and 91d of the pair of the upper and lower endless belts 91u and 91d.

### === First Modification ===

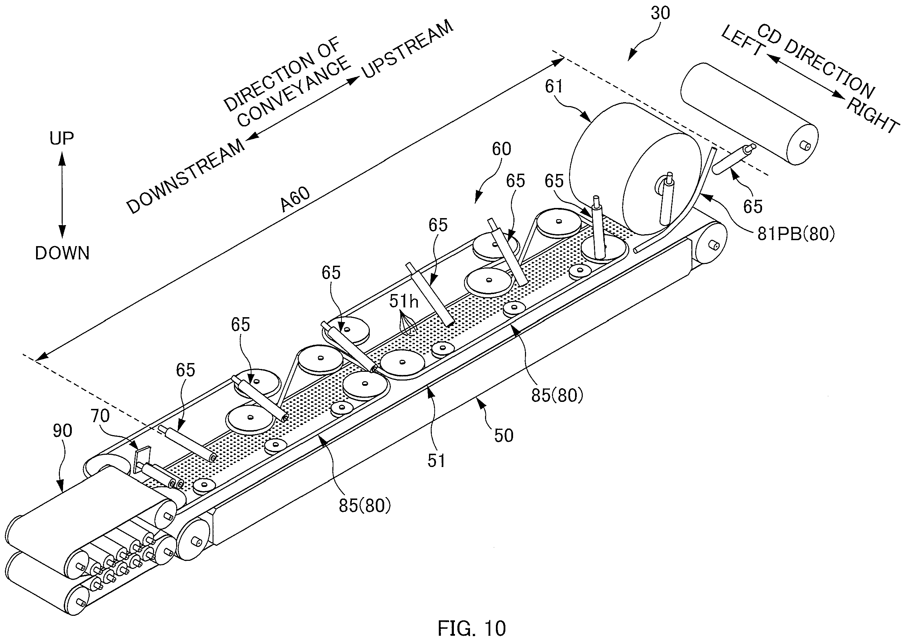

Fig. 10 is a schematic perspective view of a manufacturing apparatus 30 in a first modification. In this Fig. 10, an intermediate product 1a is not illustrated.

In the first modification, instead of the linear portion 81PL (Fig. 7) of the immovable guiding member 81 in the aforementioned embodiment, a movable guiding member 85 is disposed. Thus, the manufacturing apparatus 30 is provided with two movable guiding members 85, 85 as one example. The first modification is different from the aforementioned embodiment mainly in this respect, and other respects are mostly identical. Accordingly, the following describes mainly on this difference, and the explanations on the similar respects will be omitted.

As illustrated in Fig. 10, on the upper side of the suction belt conveyor 50, two movable guiding members 85, 85 are disposed to be aligned in the direction of conveyance. This enables the movable guiding members 85, 85 to guide the folding portion 1B over a range equal to or more than 70% of an arrangement range A60 (that is, as illustrated in Fig. 8B, a range A60 in the direction of conveyance from a position of the upstream end of the guide roller 65 positioned on the most upstream side to a position of the downstream end of the guide roller 65 positioned on the most downstream side) of the folding mechanism 60 in the direction of conveyance. Accordingly, in the state that the sliding resistance of the folding portion 1B is reduced, this folding portion 1B can be guided over a long range in the direction of conveyance.

As in the first modification, the length per movable guiding member 85 can be shortened in the case where the two movable guiding members 85, 85 are disposed so as to be aligned in the direction of conveyance compared with a case where one long movable guiding member 85 is disposed over an approximately entire length of the suction belt conveyor 50 in the direction of conveyance. Accordingly, it is possible to effectively prevent the problems such as a slack of the movable guiding member 85 that possibly occurs when the length is long, and as a result, each of the movable guiding members 85, 85 can guide the folding portion 1B of the intermediate product 1a more reliably.

### === Second Modification ==

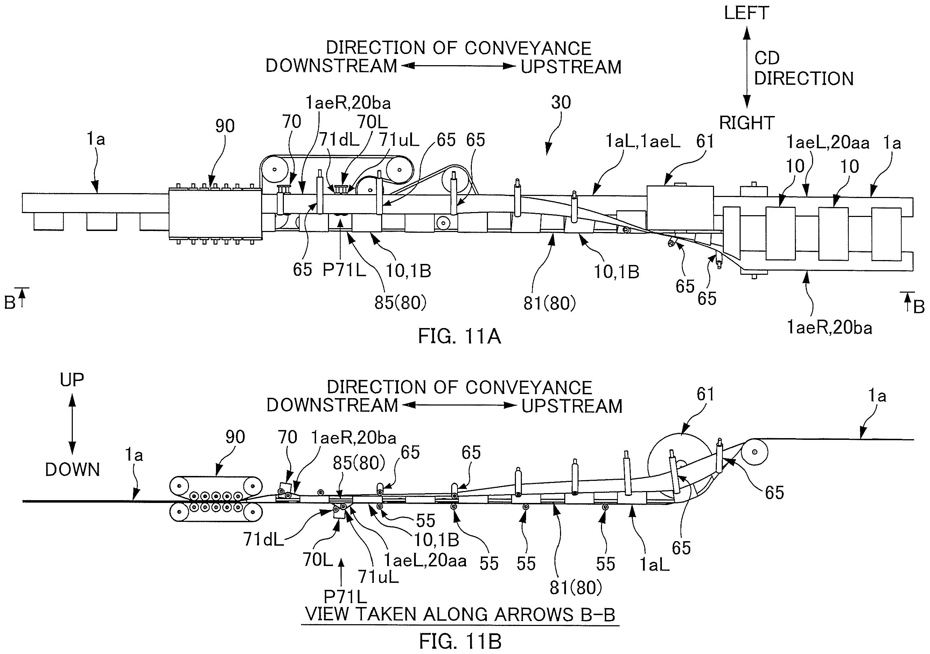

Fig. 11A is a schematic plan view of a manufacturing apparatus 30 in a second modification, and Fig. 11B is a schematic side view taken along arrows B-B in Fig. 11A.

In the aforementioned embodiment, the position adjustment mechanism 70 is disposed only to the right end portion 1aeR of the intermediate product 1a. However, the second modification is different from the aforementioned embodiment firstly in the point that a position adjustment mechanism 70L (corresponding to a second position adjustment mechanism) is additionally disposed also to the left end portion 1aeL. Further, the second modification is also different in the point that the position adjustment mechanism 70L is used as a holding mechanism so as to omit a suction belt conveyor 50. Other respects are mostly identical. Therefore, the following describes mainly on the differences, and the descriptions on the similar respects will be omitted.

In the second modification, the position adjustment mechanism 70L for the left end portion 1aeL is disposed on a position (in the example of the drawing, a position on the upstream side) near the position adjustment mechanism 70 for the right end portion 1aeR in the direction of conveyance. Then, the structure and the function of the position adjustment mechanism 70L for the left end portion 1aeL is mostly identical to the position adjustment mechanism 70 for the right end portion 1aeR. Thus, the explanation thereof will be omitted.

The position adjustment mechanism 70L for the left end portion 1aeL is also configured to function as a holding function for holding the left end portion 1aeL on the proper position in the CD direction. Accordingly, in the second modification, the suction belt conveyor 50 is omitted.

However, in the aforementioned embodiment, the suction belt conveyor 50 also has a supporting function to support an own weight of the intermediate product 1a from the lower side. Then, in order to compensate this, in the second modification, a plurality of support rollers 55, 55 ... are discretely disposed on respective positions in the direction of conveyance on the lower side of the intermediate product 1a. The support rollers 55, 55 ... abut against the left half portion 1aL of the intermediate product 1a from the lower side, thus supporting the own weight of the intermediate product 1a. The support rollers 55, 55 ... are each supported by the aforementioned panel board so as to be rotatable around rotation shafts along the CD direction. The respective support rollers 55, 55 ... may be configured as drive rollers that obtain drive rotation forces from the driving source (not illustrated) such as a servo motor to be driven to rotate, or may be configured as driven rollers that obtain rotation forces from the intermediate product 1a to co-rotate.

In the second modification, as illustrated in Fig. 11A, the movable guiding member 85 is disposed also at an arrangement position P71L of a pair of rollers 71uL and 71dL of the position adjustment mechanism 70L for the left end portion 1aeL in the direction of conveyance. That is, the movable guiding member 85 is disposed also at the position P71L (corresponding to a second predetermined position) in the direction of conveyance where this position adjustment mechanism 70L gives the external force in the CD direction to the left end portion 1aeL so as to adjust the position of the left end portion 1aeL in the CD direction. This causes the movable guiding member 85 to move downstream in the direction of conveyance together with the folding portion 1B while abutting against the folding portion 1B even at the position P71L. Accordingly, even at the position P71L, the sliding resistance that possibly occurs on the folding portion 1B of the intermediate product 1a can be reduced, and as a result, the increase in the sliding resistance, which possibly occurs in accordance with the adjustment of the position of the above-mentioned left end portion 1aeL of the intermediate product 1a, between the folding portion 1B of the intermediate product 1a and the guiding member 80 can be reduced.

Incidentally, the suction belt conveyor 50 is omitted in the second modification; however, this conveyor 50 may be disposed without being omitted in some cases. In this case, the cost of equipment slightly increases. However, the suction belt conveyor 50 that functions as the holding mechanism holds the left end portion 1aeL of the intermediate product 1a while the position adjustment mechanism 70L for the left end portion 1aeL adjusts the position of the left end portion 1aeL in the CD direction, thus ensuring the adjustment of the position with high accuracy. Along with this, the position adjustment mechanism 70 for the right end portion 1aeR adjusts the position of the right end portion 1aeR in the CD direction, and consequently, it becomes possible to place the right end portion 1aeR of the intermediate product 1a over the left end portion 1aeL with higher accuracy.

### === Other Embodiments ===

While the embodiments of the present invention are described above, the embodiments are intended for easy understanding of the present invention and are not in any way to be construed as limiting the present invention. Needless to say, the present invention may be modified and improved without departing from the scope of the invention, and equivalents thereof are also encompassed by the invention. For example, the following modifications are possible.

The above-described embodiment exemplifies the three-piece type disposable diaper 1 as an example of the absorbent article. However, the invention is not limited thereto. For example, the absorbent article may be a two-piece type disposable diaper, or may be an all-in-one type disposable diaper. Here, the two-piece type disposable diaper is a type of diaper that includes an exterior sheet in an approximately hourglass shape having a front portion, a crotch portion, and a back portion as a first component, and an absorbent main body 10 fixed to a skin side surface of this exterior sheet as a second component. Accordingly, on the aforementioned manufacturing apparatus 30, as the intermediate product of the diaper, a continuous member is conveyed in its opened state where a plurality of absorbent main bodies 10, 10 ... are placed to be fixed in a continuous direction at product pitches on a skin side surface of a continuous sheet formed of a continuous plurality of exterior sheets in a lateral direction. Then, while the intermediate product is conveyed with the continuous direction as the direction of conveyance, the manufacturing apparatus 30 folds the intermediate product in two in the CD direction perpendicular to the direction of conveyance through processes similar to the processes described in the aforementioned embodiments. On the other hand, the all-in-one type diaper is a type of diaper that includes a top sheet abutting on a skin of a target wearer and a back sheet disposed on a non-skin side in a thickness direction with respect to the top sheet, the top sheet and the back sheet each have a front portion, a crotch portion and a back portion to be formed in an approximately hourglass shape, and an absorbent body 11 is interposed between both sheets. Accordingly, on the aforementioned manufacturing apparatus 30, as the intermediate product of the diaper, a continuous member is conveyed in its opened state where a plurality of the all-in-one type diapers is aligned to be coupled in a lateral direction. Then, while the intermediate product is conveyed with the continuous direction as the direction of conveyance, the manufacturing apparatus 30 folds the intermediate product in two in the CD direction perpendicular to the direction of conveyance through processes similar to the processes described in the aforementioned embodiments.

The above-described embodiment exemplifies the pull-on disposable diaper 1 as an example of the absorbent article. However, the invention is not limited thereto. For example, the absorbent article may be a so-called tape-type disposable diaper that uses a fastening tape to be worn by the target wearer, or may be a sanitary napkin and a urine absorbing pad. That is, on the manufacturing process of the absorbent article, when a two-folding process is performed on the continuous member of the absorbent article, the manufacturing apparatus 30 and the manufacturing method of the present invention are applicable to the two-folding process.

The above-described embodiment exemplifies the round belt as an example of the movable guiding member 85. However, the invention is not limited thereto. For example, the movable guiding member may be an endless member having a flat portion such as a flat belt, or may be a string-shaped endless member such as a rope.

The above-described embodiment exemplifies the photoelectric tube as the sensor 73 associated with the position adjustment mechanism 70. However, the invention is not limited thereto. For example, a configuration that includes a CCD camera and an image processing device may be employed as the above-mentioned sensor. That is, the aforementioned actuator may be controlled in such a manner that the CCD camera takes and generates image data of the right end portion 1aeR of the intermediate product 1a at every appropriate control cycle, the image processing device performs a binarization process and the like on the image data to calculate the position of the right end portion 1aeR in the CD direction, and the calculated positional data is used as the aforementioned measurement signal to perform the control on the actuator. Incidentally, as the actuator, a device that includes a hydraulic cylinder and a conversion mechanism that converts a reciprocating operation of a piston of the hydraulic cylinder into a turning operation, a device that includes a feed screw mechanism that uses a servo motor as a driving source and a conversion mechanism that converts a reciprocating operation of the feed screw mechanism into a turning operation, or similar device can be exemplified.

In the above-mentioned embodiment, the right end portion 1aeR of the intermediate product 1a is stretched around the pair of the upper and lower rollers 71u and 71d associated with the position adjustment mechanism 70 in the S shape. However, the invention is not limited thereto. For example, the pair of the upper and lower rollers 71u and 71d may be rotated while pressing the right end portion 1aeR of the intermediate product 1a.

### [Reference Signs List]

- 1: disposable diaper (absorbent article)
- 1HB: waist opening
- 1HL: leg opening
- 1B: folding portion
- 1a: intermediate product (continuous member, two-folded member)
- 1aL: left half portion
- 1aR: right half portion
- 1aeL: left end portion (one end portion)
- 1aeR: right end portion (other end portion)
- 10: absorbent main body
- 10ea: end portion
- 10eb: end portion
- 11: absorbent body
- 11c: absorbent core
- 11r: core-wrapping sheet
- 13: front side sheet
- 15: back side sheet
- 17r: elastic member
- 20a: front band member
- 20aa: continuous body of front band member
- 20ac: central region
- 20ae: end portion
- 20b: back band member
- 20ba: continuous body of back band member
- 20bc: central region
- 20be: end portion
- 21: outer-layer sheet
- 22: inner-layer sheet
- 25: elastic string
- 30: manufacturing apparatus
- 40: two-folding device
- 50: suction belt conveyor (holding mechanism)
- 51: endless belt
- 51h: suction hole
- 55: support roller
- 60: folding mechanism
- 61: rotating drum
- 61eR: end edge
- 65: guide roller
- 70: position adjustment mechanism
- 70L: position adjustment mechanism
- 71u: roller
- 71uL: roller
- 71d: roller
- 71dL: roller
- 73: sensor
- 80: guiding member
- 81: immovable guiding member
- 81PB: curved portion
- 81PL: linear portion
- 85: movable guiding member
- 86: pulley
- 86D: pulley
- 90: pressing device
- 91u: endless belt
- 91d: endless belt
- 93: roller
- 93D: roller
- LG: leg gather
- LSG: barrier cuff
- A60: arrangement range
- A80N: unguidable section
- CL1: approximately center portion (certain portion)
- P71: arrangement position (predetermined position)
- P71L: arrangement position (second predetermined position)
- P85ED: guide termination position
- P91ST: pressing start position
- R85L: linear route

## Claims

1. A manufacturing apparatus (30) of a two-folded member associated with an absorbent article, the manufacturing apparatus (30) manufacturing two-folded members continuous in a continuous direction by folding at a folding portion (1B) a continuous member including a plurality of portions serving as absorbent articles aligned in the continuous direction so that the continuous member is folded in two in an intersecting direction, the folding portion (1B) being a predetermined portion in the intersecting direction intersecting with the continuous direction, the manufacturing apparatus (30) comprising:
a folding mechanism (60) configured to fold the continuous member at the folding portion (1B) so as to place one end portion on another end portion of the continuous member in the intersecting direction, the continuous member being conveyed in the continuous direction as a direction of conveyance;
a holding mechanism configured to hold the one end portion of the continuous member so that the one end portion is positioned at a proper position in the intersecting direction;
a guiding member (80) configured to guide the folding portion (1B) in such a manner that the guiding member (80) abuts against the continuous member to be folded from a valley side of the folding portion (1B) so as to restrict a movement of the folding portion (1B) in the intersecting direction while allowing a movement of the folding portion (1B) in the direction of conveyance; and
a position adjustment mechanism (70) configured to adjust a position of the other end portion in such a manner that the position adjustment mechanism (70) imparts an external force in the intersecting direction at a predetermined position in the direction of conveyance to the other end portion of the continuous member to be folded so as to position the other end portion at a target position in the intersecting direction,
an endless movable guiding member (85) as the guiding member (80) being disposed at least at the predetermined position in the direction of conveyance to which the external force is imparted, the endless movable guiding member (85) moving downstream in the direction of conveyance while abutting against the continuous member from the valley side of the folding portion (1B),
a non-installation region where the movable guiding member (85) is not installed existing on a conveyance path of the continuous member,
the non-installation region being provided with an immovable guiding member (81) as the guiding member (80), the immovable guiding member (81) being immovably fixed in the direction of conveyance and the intersecting direction and guiding the folding portion (1B) in the direction of conveyance,
the movable guiding member (85) being disposed downstream in the direction of conveyance with respect to the immovable guiding member (81).

2. The manufacturing apparatus (30) of a two-folded member associated with an absorbent article according to claim 1, wherein
the continuous member includes a plurality of absorbent main bodies aligned at intervals between the absorbent main bodies adjacent in the direction of conveyance, the folding portion (1B) is positioned in the absorbent main body (10),
an unguidable section (A80N) in which both the immovable guiding member (81) and the movable guiding member (85) are not able to guide the folding portion (1B) exists at a boundary position between the immovable guiding member (81) and the movable guiding member (85) in the direction of conveyance, and
the unguidable section (A80N) has a length in the direction of conveyance configured to be shorter than a length of the absorbent main body (10) in the direction of conveyance at a position of the folding portion (1B).

3. The manufacturing apparatus (30) of a two-folded member associated with an absorbent article according to claim 1 or 2, wherein
the continuous member includes a plurality of absorbent main bodies aligned at intervals between the absorbent main bodies adjacent in the direction of conveyance, the folding portion (1B) is positioned in the absorbent main body (10),
a pressing device is disposed downstream in the direction of conveyance with respect to the folding mechanism (60), the pressing device is configured to convey the continuous member in a two-folded state in the direction of conveyance while sandwiching and pressing the continuous member in a thickness direction of the continuous member, and
a length in the direction of conveyance of an interval between a guide termination position (P85ED) and a pressing start position (P91ST) is shorter than the length of the absorbent main body (10) in the direction of conveyance at the position of the folding portion (1B), the guide termination position (P85ED) being a position where the movable guiding member (85) terminates the guide of the folding portion (1B) in the direction of conveyance, the pressing start position (P91ST) being a position where the pressing device starts pressing the continuous member.

4. The manufacturing apparatus (30) of a two-folded member associated with an absorbent article according to any one of claims 1 to 3, wherein
a plurality of the movable guiding members (85) are disposed to be aligned in the direction of conveyance.

5. The manufacturing apparatus (30) of a two-folded member associated with an absorbent article according to any one of claims 1 to 4, wherein
the movable guiding member (85) guides the folding portion (1B) over a range greater than or equal to 70% of an arrangement range (A60) of the folding mechanism (60) in the direction of conveyance.

6. The manufacturing apparatus (30) of a two-folded member associated with an absorbent article according to any one of claims 1 to 5, wherein
the continuous member includes a plurality of absorbent bodies aligned at intervals between the absorbent bodies adjacent in the direction of conveyance,
a pressing device is disposed downstream in the direction of conveyance with respect to the folding mechanism (60), the pressing device configured to convey the continuous member in the two-folded state in the direction of conveyance while pressing the continuous member in a thickness direction of the continuous member,
the pressing device includes a pair of endless belts (91u, 91d) configured to be driven to go around along the direction of conveyance,
a conveyance path of the continuous member in the two-folded state is formed between the pair of the endless belts (91u, 91d), the continuous member is sandwiched and pressed by the pair of the endless belts (91u, 91d) when the continuous member passes through the conveyance path, and
an outer peripheral surface of each of the endless belts (91u, 91d) includes a portion against which the continuous member abuts, the portion has non-stickiness, and each of the endless belts (91u, 91d) has an elastic deformability in a thickness direction of the endless belts (91u, 91d), the elastic deformability being able to sandwich and press a portion without the absorbent body (11) in the continuous member.

7. The manufacturing apparatus (30) of a two-folded member associated with an absorbent article according to any one of claims 1 to 6, including:
a second position adjustment mechanism (70) configured to adjust a position of the one end portion in such a manner that the second position adjustment mechanism (70) imparts an external force in the intersecting direction to the one end portion of the continuous member at a second predetermined position in the direction of conveyance so as to position the one end portion at a target position in the intersecting direction, wherein
the movable guiding member (85) that moves downstream in the direction of conveyance while abutting against the continuous member from the valley side of the folding portion (1B) is provided also at the second predetermined position.

8. The manufacturing apparatus (30) of a two-folded member associated with an absorbent article according to claim 7, wherein
the second position adjustment mechanism (70) functions as the holding mechanism, and
a suction belt conveyor configured to hold the one end portion of the continuous member is not disposed.

9. A manufacturing method of a two-folded member associated with absorbent article, the manufacturing method manufacturing two-folded members continuous in a continuous direction by folding at a folding portion (1B) a continuous member including a plurality of portions serving as absorbent articles aligned in the continuous direction so that the continuous member is folded in two in an intersecting direction, the folding portion (1B) being a predetermined portion in the intersecting direction intersecting with the continuous direction, the manufacturing method comprising:
placing one end portion on another end portion of the continuous member in the intersecting direction by allowing a folding mechanism (60) to fold the continuous member at the folding portion (1B), the continuous member being conveyed in the continuous direction as a direction of conveyance;
holding the one end portion of the continuous member by a holding mechanism so that the one end portion is positioned at a proper position in the intersecting direction;
guiding the folding portion (1B) in such a manner that a guiding member (80) abuts against the continuous member to be folded from a valley side of the folding portion (1B) so as to restrict a movement of the folding portion (1B) in the intersecting direction while allowing a movement of the folding portion (1B) in the direction of conveyance; and
adjusting a position of the other end portion in such a manner that a position adjustment mechanism (70) imparts an external force in the intersecting direction at a predetermined position in the direction of conveyance to the other end portion of the continuous member to be folded so as to position the other end portion at a target position in the intersecting direction,
at least at the predetermined position in the direction of conveyance to which the external force is imparted, an endless movable guiding member (85) as the guiding member (80) moving downstream in the direction of conveyance while abutting against the continuous member from the valley side of the folding portion (1B),
guiding the folding portion (1B) in the direction of conveyance in a non-installation region, wherein the non-installation region is a region where the movable guiding member (85) is not installed existing on a conveyance path of the continuous member,
the non-installation region being provided with an immovable guiding member (81) as the guiding member (80), the immovable guiding member (81) being immovably fixed in the direction of conveyance and the intersecting direction and guiding the folding portion (1B) in the direction of conveyance,
the movable guiding member (85) being disposed downstream in the direction of conveyance with respect to the immovable guiding member (81).

## Patentansprüche

1. Herstellungsvorrichtung (30) eines zweifach gefalteten Elements, das einem absorbierenden Artikel zugeordnet ist, wobei die Herstellungsvorrichtung (30) kontinuierlich in einer kontinuierlichen Richtung zweifach gefaltete Elemente herstellt, durch Falten an einem Faltabschnitt (1B) eines kontinuierlichen Elements, das eine Vielzahl von Abschnitten umfasst, die als absorbierende Artikel dienen, die in der kontinuierlichen Richtung ausgerichtet sind, so dass das kontinuierliche Element in zwei Teile in einer Schnittrichtung gefaltet wird, wobei der Faltabschnitt (1B) ein vorbestimmter Abschnitt in der Schnittrichtung ist, die sich mit der kontinuierlichen Richtung schneidet, wobei die Herstellungsvorrichtung (30) umfasst:
einen Faltmechanismus (60), der zum Falten des kontinuierliche Elements an dem Faltabschnitt (1B) ausgebildet ist, um einen Endabschnitt an einem anderen Endabschnitt des kontinuierlichen Elements in der Schnittrichtung zu platzieren, wobei das kontinuierliche Element in der kontinuierlichen Richtung als eine Förderrichtung gefördert wird;
einen Haltemechanismus, der zum Halten des Endabschnitts des kontinuierlichen Elements ausgebildet ist, sodass der eine Endabschnitt an einer geeigneten Position in der Schnittrichtung angeordnet ist;
ein Führungselement (80), das zum Führen des Faltabschnitt (1B) ausgebildet ist, derart, dass das Führungselement (80) von einer Talseite des Faltabschnitts (1B) aus gegen das zu faltende kontinuierliche Element anliegt, um eine Bewegung des Faltabschnitts (1B) in der Schnittrichtung einzuschränken, während eine Bewegung des Faltabschnitts (1B) in der Förderrichtung ermöglicht wird; und
einen Positionseinstellmechanismus (70), der zum Einstellen einer Position des anderen Endabschnitts ausgebildet ist, sodass der Positionseinstellmechanismus (70) eine externe Kraft in der Schnittrichtung an einer vorbestimmten Position in der Transportrichtung zu dem anderen Endabschnitt des zu faltenden kontinuierlichen Elements überträgt, um den anderen Endabschnitt an einer Zielposition in der Schnittrichtung anzuordnen,
ein endlos bewegliches Führungselement (85) als Führungselement (80), das mindestens an der vorbestimmten Position in der Förderrichtung angeordnet ist, der die äußere Kraft zugeführt wird, wobei sich das endlos bewegliche Führungselement (85) stromabwärts in Förderrichtung bewegt, während es von der Talseite des Faltabschnitts (1B) an das kontinuierliche Element angrenzt,
einen Nicht-Einbaubereich, in dem das bewegliche Führungselement (85) nicht installiert ist, der auf einer Förderstrecke des kontinuierlichen Elements vorhanden ist,
wobei der Nicht-Einbau-Bereich mit einem unbeweglichen Führungselement (81) als das Führungselement (80) versehen ist, wobei das unbewegliche Führungselement (81) in Förderrichtung und Schnittrichtung unbeweglich befestigt ist und den Faltabschnitt (1B) in Förderrichtung führt,
wobei das bewegliche Führungselement (85) stromabwärts in Förderrichtung in Bezug auf das unbewegliche Führungselement (81) angeordnet ist.

2. Die Herstellungsvorrichtung (30) eines zweifach gefalteten Elements, das mit einem absorbierenden Artikel gemäß Anspruch 1 verbunden ist, wobei
das kontinuierliche Element eine Vielzahl von absorbierenden Hauptkörpern umfasst, die in Abständen zwischen den absorbierenden Hauptkörpern ausgerichtet sind, die in Förderrichtung benachbart sind, wobei der Faltabschnitt (1B) in dem absorbierenden Hauptkörper (10) angeordnet ist,
einen nicht führbaren Abschnitt (A80N), in dem sowohl das unbewegliche Führungselement (81) als auch das bewegliche Führungselement (85) nicht in der Lage sind, den Faltabschnitt (1B) zu führen, an einer Grenzposition zwischen dem unbeweglichen Führungselement (81) und dem beweglichen Führungselement (85) in Förderrichtung, vorhanden ist, und
der nicht führbare Abschnitt (A80N) eine Länge in Förderrichtung aufweist, die so ausgebildet ist, dass sie kürzer ist als eine Länge des absorbierenden Hauptkörpers (10) in Förderrichtung an einer Position des Faltabschnitts (1B).

3. Die Herstellungsvorrichtung (30) eines zweifach gefalteten Elements, das mit einem absorbierenden Artikel gemäß Anspruch 1 oder 2 verbunden ist, wobei
das kontinuierliche Element eine Vielzahl von absorbierenden Hauptkörpern beinhaltet, die in Abständen zwischen den absorbierenden Hauptkörpern ausgerichtet sind, die in Förderrichtung benachbart sind, wobei der Klappabschnitt (1B) in dem absorbierenden Hauptkörper (10) angeordnet ist,
eine Pressvorrichtung stromabwärts in Förderrichtung in Bezug auf den Faltmechanismus (60) angeordnet ist, wobei die Pressvorrichtung ausgebildet ist, um das kontinuierliche Element in einem zwei gefalteten Zustand in Förderrichtung zu fördern, während sie das kontinuierliche Element in einer Dickenrichtung des kontinuierlichen Elements sandwichartig umgibt und presst, und
eine Länge in Förderrichtung eines Intervalls zwischen einer Führungsendposition (P85ED) und einer Pressstartposition (P91ST) kürzer ist als die Länge des absorbierenden Hauptkörpers (10) in Förderrichtung an der Position des Klappabschnitts (1B), wobei die Führungsendposition (P85ED) eine Position ist, in der das bewegliche Führungselement (85) die Führung des Faltabschnitts (1B) in Förderrichtung beendet, wobei die Pressstartposition (P91ST) eine Position ist, in der die Pressvorrichtung mit dem Pressen des kontinuierlichen Elements beginnt.

4. Die Herstellungsvorrichtung (30) eines zweifach gefalteten Elements, das mit einem absorbierenden Artikel gemäß einem der Ansprüche 1 bis 3 verbunden ist, wobei
eine Vielzahl der beweglichen Führungselemente (85) angeordnet sind, um in Förderrichtung ausgerichtet zu sein.

5. Die Herstellungsvorrichtung (30) eines zweifach gefalteten Elements, das mit einem absorbierenden Artikel gemäß einem der Ansprüche 1 bis 4 verbunden ist, wobei
das bewegliche Führungselement (85) den Faltabschnitt (1B) über einen Bereich führt, der größer oder gleich 70% eines Anordnungsbereichs (A60) des Faltmechanismus (60) in Förderrichtung ist.

6. Die Herstellungsvorrichtung (30) eines zweifach gefalteten Elements, das mit einem absorbierenden Artikel gemäß einem der Ansprüche 1 bis 5 verbunden ist, wobei
das kontinuierliche Element eine Vielzahl von absorbierenden Körpern umfasst, die in Abständen zwischen den absorbierenden Körpern in Förderrichtung benachbart angeordnet sind,
eine Pressvorrichtung stromabwärts in Förderrichtung in Bezug auf den Faltmechanismus (60) angeordnet ist, wobei die Pressvorrichtung ausgebildet ist, um das kontinuierliche Element im zweifach gefalteten Zustand in Förderrichtung zu fördern, während das kontinuierliche Element in eine Dickenrichtung des kontinuierlichen Elements gedrückt wird,
die Pressvorrichtung ein Paar Endlosbänder (91u, 91d) umfasst, die ausgebildet sind, um angetrieben zu werden, um entlang der Förderrichtung umherzugehen,
ein Förderweg des kontinuierlichen Elements im zweifach gefalteten Zustand zwischen dem Paar der Endlosbänder (91u, 91d) ausgebildet wird, wobei das kontinuierliche Element durch das Paar der Endlosbänder (91u, 91d) sandwichartig umgeben ist und gepresst wird, wenn das kontinuierliche Element den Förderweg durchläuft, und
eine äußere Umfangsfläche jedes der Endlosbänder (91u, 91d) einen Abschnitt umfasst, an dem das kontinuierliche Element anliegt, wobei der eine Abschnitt eine Antihaftwirkung aufweist und jedes der Endlosbänder (91u, 91d) eine elastische Verformbarkeit in einer Dickenrichtung der Endlosbänder (91u, 91d) aufweist, wobei die elastische Verformbarkeit in der Lage ist, einen Abschnitt ohne den absorbierenden Körper (11) in dem kontinuierlichen Element sandwichartig zu umgeben zu pressen.

7. Die Herstellungsvorrichtung (30) eines zweifach gefalteten Elements, das mit einem absorbierenden Artikel gemäß einem der Ansprüche 1 bis 6 verbunden ist, umfassend:
einen zweiten Positionseinstellmechanismus (70), der ausgebildet ist, um eine Position des einen Endabschnitts so einzustellen, dass der zweite Positionseinstellmechanismus (70) dem einen Endabschnitt des kontinuierlichen Elements an einer zweiten vorbestimmten Position in Förderrichtung eine externe Kraft in der Schnittrichtung aufprägt, um den einen Endabschnitt an einer Zielposition in der Schnittrichtung anzuordnen, wobei
das bewegliche Führungselement (85), das sich stromabwärts in Förderrichtung bewegt, während es von der Talseite des Faltabschnitts (1B) aus an das kontinuierliche Element angrenzt, auch an der zweiten vorbestimmten Position vorgesehen ist.

8. Die Herstellungsvorrichtung (30) eines zweifach gefalteten Elements, das mit einem absorbierenden Artikel gemäß Anspruch 7 verbunden ist, wobei
der zweite Positionseinstellmechanismus (70) als der Haitemechanismus fungiert, und
ein Saugbandförderer, der ausgebildet ist, um den einen Endabschnitt des kontinuierlichen Elements zu halten, nicht angeordnet ist.

9. Herstellungsverfahren eines zweifach gefalteten Elements, das mit einem absorbierenden Artikel verbunden ist, wobei das Herstellungsverfahren zweifach gefaltete Elemente kontinuierlich herstellt, in einer kontinuierlichen Richtung durch Falten an einem Faltabschnitt (1B) eines kontinuierlichen Elements mit einer Vielzahl von Abschnitten, die als absorbierende Artikel dienen, die in der kontinuierlichen Richtung ausgerichtet sind, so dass das kontinuierliche Element in einer Schnittrichtung zweifach gefaltet wird, wobei der Faltabschnitt (1B) ein vorbestimmter Abschnitt in der Schnittrichtung ist, der sich mit der kontinuierlichen Richtung schneidet, wobei das Herstellungsverfahren umfasst:
Platzieren eines Endabschnitts auf einem anderen Endabschnitt des kontinuierlichen Elements in der Schnittrichtung, indem ein Faltmechanismus (60) das kontinuierliche Element an dem Klappabschnitt (1B) falten kann, wobei das kontinuierliche Element in der kontinuierlichen Richtung als Förderrichtung gefördert wird;
Halten des einen Endabschnitts des kontinuierlichen Elements durch einen Haltemechanismus, so dass der eine Endabschnitt an einer geeigneten Position in Schnittrichtung angeordnet ist;
Führen des Faltabschnitts (1B) derart, dass ein Führungselement (80) von einer Talseite des Faltabschnitts (1B) aus gegen das zu faltende Endloselement anliegt, um eine Bewegung des Faltabschnitts (1B) in der Schnittrichtung einzuschränken, während eine Bewegung des Faltabschnitts (1B) in Förderrichtung ermöglicht wird; und
Einstellen einer Position des anderen Endabschnitts derart, dass ein Positionseinstellmechanismus (70) eine externe Kraft in der Schnittrichtung an einer vorbestimmten Position in der Förderrichtung an den anderen Endabschnitt des zu faltenden kontinuierlichen Elements überträgt, um den anderen Endabschnitt an einer Zielposition in der Schnittrichtung anzuordnen,
wenigstens an der vorbestimmten Position in Förderrichtung, an der die äußere Kraft aufgetragen wird, ein endlos bewegliches Führungselement (85) als das Führungselement (80), das sich in Förderrichtung stromabwärts bewegt, während es von der Talseite des Klappabschnitts (1B) an dem durchgehende Element anliegt,
Führen des Faltabschnitts (1B) in Förderrichtung in einem Nicht-Einbaubereich, wobei der Nicht-Einbaubereich ein Bereich ist, in dem das bewegliche Führungselement (85) nicht angeordnet ist, das auf einem Förderweg des kontinuierlichen Elements vorhanden ist,
wobei der Nicht-Einbaubereich mit einem unbeweglichen Führungselement (81) als das Führungselement (80) versehen ist, wobei das unbewegliche Führungselement (81) in Förderrichtung und Schnittrichtung unbeweglich befestigt ist und den Faltabschnitt (1B) in der Förderrichtung führt,
wobei das bewegliche Führungselement (85) stromabwärts in Förderrichtung in Bezug auf das unbewegliche Führungselement (81) angeordnet ist.

## Revendications

1. Appareil de production (30) d'un élément plié en deux associé à un article absorbant, l'appareil de production (30) produisant des éléments pliés en deux continus dans une direction continue en pliant au niveau d'une portion de pliage (1B) un élément continu comportant une pluralité de portions servant d'articles absorbants alignés dans la direction continue de sorte que l'élément continu soit plié en deux dans une direction de croisement, la portion de pliage (1B) étant une portion prédéterminée dans la direction de croisement croisant la direction continue, l'appareil de production (30) comprenant :
un mécanisme de pliage (60) configuré pour plier l'élément continu au niveau de la portion de pliage (1B) de façon à placer une portion d'extrémité sur une autre portion d'extrémité de l'élément continu dans la direction de croisement, l'élément continu étant transporté dans la direction continue en tant que direction de transport ;
un mécanisme de maintien configuré pour maintenir la portion d'extrémité de l'élément continu de sorte que la portion d'extrémité soit positionnée à une position correcte dans la direction de croisement ;
un élément de guidage (80) configuré pour guider la portion de pliage (1B) de manière à ce que l'élément de guidage (80) bute contre l'élément continu à plier depuis un côté de creux de la portion de pliage (1B) de façon à limiter un déplacement de la portion de pliage (1B) dans la direction de croisement tout en permettant un déplacement de la portion de pliage (1B) dans la direction de transport ;
et
un mécanisme de réglage de position (70) configuré pour régler une position de l'autre portion d'extrémité de manière à ce que le mécanisme de réglage de position (70) communique une force externe dans la direction de croisement à une position prédéterminée dans la direction de transport à l'autre portion d'extrémité de l'élément continu à plier de façon à positionner l'autre portion d'extrémité à une position cible dans la direction de croisement,
un élément de guidage mobile sans fin (85) en tant qu'élément de guidage (80) qui est disposé au moins à la position prédéterminée dans la direction de transport à laquelle la force externe est communiquée, l'élément de guidage mobile sans fin (85) se déplaçant en aval dans la direction de transport tout en butant contre l'élément continu depuis le côté de creux de la portion de pliage (1B).
une région de non-installation où l'élément de guidage mobile (85) n'est pas installé existant sur un trajet de transport de l'élément continu,
la région de non-installation étant pourvue d'un élément de guidage immobile (81) en tant qu'élément de guidage (80), l'élément de guidage immobile (81) étant fixé immobile dans la direction de transport et la direction de croisement et guidant la portion de pliage (1B) dans la direction de transport,
l'élément de guidage mobile (85) étant disposé en aval dans la direction de transport par rapport à l'élément de guidage immobile (81).

2. Appareil de production (30) d'un élément plié en deux associé à un article absorbant selon la revendication 1, dans lequel
l'élément continu comporte une pluralité de corps principaux absorbants alignés à des intervalles entre les corps principaux absorbants adjacents dans la direction de transport, la portion de pliage (1B) est positionnée dans le corps principal absorbant (10),
une section non guidable (A80N), dans laquelle l'élément de guidage immobile (81) et l'élément de guidage mobile (85) ne sont tous deux pas capables de guider la portion de pliage (1B), existe à une position limite entre l'élément de guidage immobile (81) et l'élément de guidage mobile (85) dans la direction de transport, et
la section non guidable (A80N) a une longueur dans la direction de transport configurée pour être plus courte qu'une longueur du corps principal absorbant (10) dans la direction de transport à une position de la portion de pliage (1B).

3. Appareil de production (30) d'un élément plié en deux associé à un article absorbant selon la revendication 1 ou 2, dans lequel
l'élément continu comporte une pluralité de corps principaux absorbants alignés à des intervalles entre les corps principaux absorbants adjacents dans la direction de transport, la portion de pliage (1B) est positionnée dans le corps principal absorbant (10),
un dispositif de pression est disposé en aval dans la direction de transport par rapport au mécanisme de pliage (60), le dispositif de pression est configuré pour transporter l'élément continu dans un état plié en deux dans la direction de transport tout en enserrant et en pressant l'élément continu dans une direction d'épaisseur de l'élément continu, et
une longueur dans la direction de transport d'un intervalle entre une position de fin de guidage (P85ED) et une position de commencement de pression (P91ST) est plus courte que la longueur du corps principal absorbant (10) dans la direction de transport à la position de la portion de pliage (1B), la position de fin de guidage (P85ED) étant une position où l'élément de guidage mobile (85) finit de guider la portion de pliage (1B) dans la direction de transport, la position de commencement de pression (P91ST) étant une position où le dispositif de pression commence à presser l'élément continu.

4. Appareil de production (30) d'un élément plié en deux associé à un article absorbant selon l'une quelconque des revendications 1 à 3, dans lequel
une pluralité des éléments de guidage mobiles (85) sont disposés pour être alignés dans la direction de transport.

5. Appareil de production (30) d'un élément plié en deux associé à un article absorbant selon l'une quelconque des revendications 1 à 4, dans lequel
l'élément de guidage mobile (85) guide la portion de pliage (1B) sur une plage supérieure ou égale à 70 % d'une plage d'agencement (A60) du mécanisme de pliage (60) dans la direction de transport.

6. Appareil de production (30) d'un élément plié en deux associé à un article absorbant selon l'une quelconque des revendications 1 à 5, dans lequel
l'élément continu comporte une pluralité de corps absorbants alignés à des intervalles entre les corps absorbants adjacents dans la direction de transport,
un dispositif de pression est disposé en aval dans la direction de transport par rapport au mécanisme de pliage (60), le dispositif de pression étant configuré pour transporter l'élément continu dans l'état plié en deux dans la direction de transport tout en pressant l'élément continu dans une direction d'épaisseur de l'élément continu,
le dispositif de pression comporte une paire de courroies sans fin (91u, 91d) configurées pour être entraînées à circuler le long de la direction de transport,
un trajet de transport de l'élément continu dans l'état plié en deux est formé entre la paire de courroies sans fin (91u, 91d), l'élément continu est enserré et pressé par la paire de courroies sans fin (91u, 91d) lorsque l'élément continu passe par le trajet de transport,
une surface périphérique extérieure de chacune des courroies sans fin (91u, 91d) comporte une portion contre laquelle bute l'élément continu, la portion a un caractère non collant, et chacune des courroies sans fin (91u, 91d) a une déformabilité élastique dans une direction d'épaisseur des courroies sans fin (91u, 91d), la déformabilité élastique étant capable d'enserrer et de presser une portion sans le corps absorbant (11) dans l'élément continu.

7. Appareil de production (30) d'un élément plié en deux associé à un article absorbant selon l'une quelconque des revendications 1 à 6, comportant :
un second mécanisme de réglage de position (70) configuré pour régler une position de la portion d'extrémité de manière à ce que le second mécanisme de réglage de position (70) communique une force externe dans la direction de croisement à la portion d'extrémité de l'élément continu à une seconde position prédéterminée dans la direction de transport de façon à positionner la portion d'extrémité à une position cible dans la direction de croisement, dans lequel
l'élément de guidage mobile (85) qui se déplace en aval dans la direction de transport tout en butant contre l'élément continu depuis le côté de creux de la portion de pliage (1B) est également prévu à la seconde position prédéterminée.

8. Appareil de production (30) d'un élément plié en deux associé à un article absorbant selon la revendication 7, dans lequel
le second mécanisme de réglage de position (70) fonctionne en tant que mécanisme de maintien, et
un transporteur à courroie à aspiration configuré pour maintenir la portion d'extrémité de l'élément continu n'est pas disposé.

9. Procédé de production d'un élément plié en deux associé à un article absorbant, le procédé de production produisant des éléments pliés en deux continus dans une direction continue en pliant au niveau d'une portion de pliage (1B) un élément continu comportant une pluralité de portions servant d'articles absorbants alignés dans la direction continue de sorte que l'élément continu soit plié en deux dans une direction de croisement, la portion de pliage (1B) étant une portion prédéterminée dans la direction de croisement croisant la direction continue, le procédé de production comprenant :
le placement d'une portion d'extrémité sur une autre portion d'extrémité de l'élément continu dans la direction de croisement en permettant à un mécanisme de pliage (60) de plier l'élément continu au niveau de la portion de pliage (1B), l'élément continu étant transporté dans la direction continue en tant que direction de transport ;
le maintien de la portion d'extrémité de l'élément continu par un mécanisme de maintien de sorte que la portion d'extrémité soit positionnée à une position correcte dans la direction de croisement ;
le guidage de la portion de pliage (1B) de manière à ce qu'un élément de guidage (80) bute contre l'élément continu à plier depuis un côté de creux de la portion de pliage (1B) de façon à limiter un déplacement de la portion de pliage (1B) dans la direction de croisement tout en permettant un déplacement de la portion de pliage (1B) dans la direction de transport ; et
le réglage d'une position de l'autre portion d'extrémité de manière à ce qu'un mécanisme de réglage de position (70) communique une force externe dans la direction de croisement à une position prédéterminée dans la direction de transport à l'autre portion d'extrémité de l'élément continu à plier de façon à positionner l'autre portion d'extrémité à une position cible dans la direction de croisement,
au moins à la position prédéterminée dans la direction de transport à laquelle la force externe est communiquée, un élément de guidage mobile sans fin (85) en tant qu'élément de guidage (80) se déplaçant en aval dans la direction de transport tout en butant contre l'élément continu depuis le côté de creux de la portion de pliage (1B),
le guidage de la portion de pliage (1B) dans la direction de transport dans une région de non-installation, dans lequel la région de non-installation est une région où l'élément de guidage mobile (85) n'est pas installé existant sur un trajet de transport de l'élément continu,
la région de non-installation étant pourvue d'un élément de guidage immobile (81) en tant qu'élément de guidage (80), l'élément de guidage immobile (81) étant fixé immobile dans la direction de transport et la direction de croisement et guidant la portion de pliage (1B) dans la direction de transport,
l'élément de guidage mobile (85) étant disposé en aval dans la direction de transport par rapport à l'élément de guidage immobile (81).
